# EUROPEAN PATENT APPLICATION

(11) **EP 4 230 749 A1**
(43) Date of publication of application: **23.08.2023**
(21) Application number: 22305188.9
(22) Date of filing: 21.02.2022
(51) Int. Cl.: C12Q 1/6883

(54) **IN VITRO METHOD FOR PREDICTING A RISK OF POST-CHIKUNGUNYA CHRONIC INFLAMMATORY JOINT DISEASE**

(71) Applicant: BIOMÉRIEUX, 69280 Marcy L'Etoile (FR); Universidade de São Paulo - USP, 05508-220 São Paulo (BR)
(72) Inventor: SEVERO RAMUNDO, Mariana, 01306-010 SÃO PAULO (BR); CERDEIRA SABINO, Ester, 01310 000 SÃO PAULO (BR); DOS SANTOS LÁZARI, Carolina, 02022-020 SÃO PAULO (BR); MANULI, Erika Regina, 04153-001 SÃO PAULO (BR); TEN CATEN, Felipe, ATLANTA, 30324 (US); FARREL CÔRTES, Marina, 05409-002 SÃO PAULO (BR); PARANHOS-BACCALA, Glaucia, 69003 LYON (FR)
(74) Representative: Plasseraud IP

(57) **Abstract**

The invention relates to the field of *in vitro* diagnosis or prognosis. The invention provides *in vitro* methods and kit for predicting the risk of developing Post-Chikungunya Chronic Inflammatory Joint Disease (pCHIKV-CIJD) in a subject. The method comprises a step of evaluating the expression of one or more biomarkers in a biological sample obtained from said subject, for example a blood sample, wherein said one or more biomarkers are selected among specific long non-coding RNAs, and wherein a higher expression than control expression value indicates a risk of developing pCHIKV-CIJD.

## Description

### FIELD OF THE INVENTION

The invention relates to the field of *in vitro* diagnosis or prognosis. The invention provides *in vitro* methods for predicting the risk of developing Post-Chikungunya Chronic Inflammatory Joint Disease (pCHIKV-CIJD) in a subject. More specifically, the method comprises detecting and/or evaluating the expression of one or more biomarkers selected from specific long non-coding RNA sequences, in a biological sample obtained from said subject, for example a blood sample, aiming to forecast the development of chronic presentations of disease.

### BACKGROUND

Chikungunya virus (CHIKV) is an RNA virus, member of the *Togaviridae* family and the *Alphavirus* genus. It was first described in 1952, in Tanzania, and was then responsible for outbreaks in Africa and Southeast Asia. By the 2000s, CHIKV has caused epidemics of unprecedented magnitude. As of 2013, it has gained attention once it has been detected and spread rapidly across the Caribbean and Central and South America, where more than 1.4 million cases have been reported, both suspected and confirmed, with transmission in 50 countries, including Brazil^{1,2}. This virus, transmitted mainly by *Aedes* species of mosquitoes, causes a vector-borne disease characterized by fever associated with joint edema and pain, which occurs after a mean incubation period of three days after an infected mosquito bite³. Although most patients have symptoms resolution after a period of about seven days, 25%-40% of them have more severe implications including neurological manifestations chronic arthralgia⁴

Chronic Chikungunya fever is usually defined when joint pain is persistent for over three months, with or without other signs and symptoms. The association of chronic pain with at least one sign of arthritis, mostly in more than four joints, that lasts more than six weeks and does not meet criteria for rheumatoid arthritis or spondyloarthritis is known as post-Chikungunya chronic inflammatory joint disease (pCHIKV-CIJD). Long-term pCHIKV-CIJD leads to a reduction in the patient's quality of life, impairing their mobility. Thus, biomarkers that could predict patients who might have pCHIKV-CIJD could lead to a gain in the quality of life of these patients, as it would allow early medical interventions that could prevent or reduce the damage caused by CHIKV disease.

Despite the importance of the disease, few studies aim to find biomarkers of disease progression Lisa Ng and co-workers⁵ assessed the serum levels of 30 inflammatory mediators and growth factors in samples from patients with infection or severe disease who were affected during the first outbreak of CHIKV in Singapore. After statistical analyses, the authors reported that an increase in IL-1β, IL-6 and a decrease in RANTES was associated with severity.

Years later, in 2016, a study of Sepúlveda-Delgado and co-workers⁶ set out to evaluate biomarkers capable of predicting clinical progression to chronic joint disease after CHIKV. The authors performed a prospective study in which patients with CHIKV were followed for 1 year with monthly visits. Patients with pCHIKV-CIJD were those who persisted with symptoms 3 months after infection. Of the 10 patients included, 6 had subacute infection and/or chronic disease. During follow-up, IL-6 was statistically different in patients with subacute and chronic symptoms compared to those who resolved in the acute phase (p < 0.05) and therefore was related to chronicity of articular symptoms and could be used as predictor of CHIKV-induced arthritis.

Subsequently, elevated ferritin levels were associated with pCHIKV-CIJD in a cohort of 116 patients diagnosed with CHIKV in a disease outbreak reported in Curaçao⁷. More recently, in 2020, a study of 106 patients reported that there was no significance in comparing levels of inflammatory biomarkers, including ferritin, in the presence of moderate chronic joint disease in the study population⁸.

In 2019, a study⁹ was conducted in which the authors compared the expression of 12 cytokines/chemokines and matrix metalloproteinases (MMP)-1 and MMP-3 using enzyme-linked immunosorbent assays (ELISAs) in three groups of well-established patients infected during an outbreak occurred between 2008 and 2009 in Thailand. Subjects comprised 30 patients who had acute illness and were fully recovered, 30 healthy controls who had never had CHIKV, and 63 patients with persistent arthralgia and who still had joint symptoms 56 to 60 months after the outbreak occurred. The authors noted that TNF-α, IL-6, IL-8, and MCP-1 may play a role in persistent arthralgia or chronic disease through activation of MMP-1 and MMP-3. The increase in TNF-α, IL-12, and MCP-1 levels (and the tendency towards an increase in IFN-γ, IL-1β, IL-6, and IL-8 levels) in patients with severe pain compared with patients with non-severe pain suggests the role of these inflammatory markers in chronic disease and severity of the disease.

Still considering the inflammatory markers, the serum levels of IL-17A, IL-21, IL-22, IL-27, IL-29, and TGF-β were evaluated with specific ELISA kits in 45 patients older than 18 years with musculoskeletal manifestations of CHIKV in the subacute and chronic phase of the disease and 49 healthy controls¹⁰. Serum levels of IL-27 were significantly higher in patients with CHIKV disease than in controls and correlated with persistence of CHIKV disease and arthralgia. IL-17A, IL-29, and TGF-β were also significantly elevated in patients, and, IL-17A correlated with arthritis, which might indicate the inflammatory nature of Chikungunya infection in patients with joint symptoms and roles of those cytokines in the disease's pathophysiology.

In 2020, a Brazilian group¹¹ compared healthy individuals with individuals suffering from subacute disease and chronic musculoskeletal manifestations and observed that the latter presented increased levels of galectin-9 (GAL-9) in the chronic phase of the disease. GAL-9 levels were correlated with the intensity of morning stiffness and were associated with its duration. The authors suggested that despite a link between innate and adaptive immunity, and its relation to the chronicity of viral diseases, galectin-9 may be an interesting therapeutic target.

Finally, a systematic review and meta-analysis aimed to evaluate existing biomarkers in the literature that can predict the severity and chronicity of CHIKV¹².. Seventeen articles were evaluated and, after analysis, several biomarkers were associated with disease severity, such as increased levels of IL-6, IP-10, IL-1b, MIG, MCP-1, and reduced levels of RANTES and IL- 8. Other markers were associated to chronification, as increased levels of IL-6, TNF-α, MCP-1, IL-12, INF-α, IL-13, INF-γ, GM-CSF, CRP, IL-1a, IL-15, Factor VII, IP-10, IL-10, IL-4, IL-1RA, IL-8, MIP-1α, MIP-1β, ferritin, MIG, ESR, NO, malondialdehyde, and low levels of RANTES, ferritin, eotaxin , HGF, IL-27, IL-17A, IL-29, TGF-β, IL-10, and thiols. IL-6, CRP and TNF-α were included in the meta-analysis to assess the relationship with chronicity, although no statistical significance was found.

In spite of all these efforts, to date, there is still no precise diagnostic tool to identify patients having CHIKV disease, in particular during the acute or subacute phase of CHIKV disease, who are at increased risk of developing of Chronic Inflammatory Joint Disease Post-Chikungunya (pCHIKV-CIJD). The identification and validation of predictive biomarkers of these unfavorable outcomes would allow a more accurate assessment of risk in different populations, enabling better medical care and more rational targeting of resources, reducing the great impact of the CHIKV disease on the health system in tropical countries, such as Brazil.

### SUMMARY

A first object of the disclosure relates to an *in vitro* method for predicting the risk of developing Post-Chikungunya Chronic Inflammatory Joint Disease (pCHIKV-CIJD) in a subject, said method comprising detecting and/or evaluating the expression of one or more biomarkers in a biological sample obtained from said subject, for example a blood sample, wherein said one or more biomarkers are selected from the following long non-coding RNA sequences identified below as SEQ ID NOs1-4 or a variant sequence thereof having at least 95% identity with one of the sequences identified in SEQ ID NOs 1-4:

| Lnc-RNA# | Reference | SEQ ID NO: |
|---|---|---|
| LNCRNA1 | AC104561.4 | 1 |
| LNCRNA2 | AP000224.1 | 2 |
| LNCRNA3 | AC098484.1 | 3 |
| LNCRNA4 | AC124283.1 | 4 |

Another object of the disclosure relates to a kit comprising means for amplifying and/or detecting at least one sequence selected from the long non-coding RNA sequences identified in SEQ ID NOs:1-4, wherein the amplification and/or detection means allow the amplification, the detection and/or the quantification, of 1, 2, 3 or all 4 long non-coding RNA sequences in a biological sample.

Another object of the disclosure relates to a method of treating or preventing Chronic Inflammatory Joint Disease Post-Chikungunya (pCHIKV-CIJD) in a subject in need thereof, said method comprising determining the risk of developing pCHIKV-CIJD in a subject as described in detail below, and administering a suitable therapeutic or prophylactic treatment of pCHIKV-CIJD in said subject selected as presenting a risk of developing pCHIKV-CIJD.

### DETAILED DESCRIPTION

As used herein, a subject having Post-Chikungunya Chronic Inflammatory Joint Disease (also referred as pCHIKV-CIJD) is a subject with persistent joint pain following confirmed CHIKV infection, with at least one objective sign of arthritis, mostly in more than four joints, that lasts more than 6 weeks and does not meet the criteria for rheumatoid arthritis or spondyloarthritis. Objective signs of arthritis (edema, erythema, heat, limitation of movement range) may be present during physical examination but can be confirmed by an accurate tool such as ultrasonography. This definition of pCHIKV-CIJD is also in accordance with the Recommendations of the Brazilian Society of Rheumatology for diagnosis and treatment of Chikungunya fever¹³.

The term "patient" or "subject" which is used herein interchangeably refers to a human being, including for example a man or a woman, that has been diagnosed with chikungunya fever, advantageously confirmed by RT-PCR or IgM detection by ELISA, who is therefore at risk of developing pCHIKV-CIJD.

In specific embodiments, the subject to be tested for assessing risk of developing pCHIKV-CIJD is within the acute phase or subacute phase of chikungunya fever and exhibits symptoms of the acute phase or subacute phase.

As used herein, a subject is described as being in the "acute" phase of Chikungunya infection if the Chikungunya infection is in its early phases, for example, within day 2 to day 10 of infection.

As used herein, a subject is described as being in the "subacute" phase of Chikungunya infection if the Chikungunya infection is from day 11 to day 30, 60 or 90 of infection. There may be absence of fever and persistence or worsening of arthralgia. To be classified as a chronic phase of the disease, symptoms must persist for more than three months after the onset of the disease.

Accordingly, in certain embodiments, the method further comprises determining a prognosis of the subject based on the expression level of one or more biomarkers selected from the following long non-coding RNA sequences identified below as SEQ ID NOs 1-4 or a variant sequence thereof having at least 95% identity with one of the sequences identified in SEQ ID NOs1-4:

| Lnc-RNA# | Reference | SEQ ID NO |
|---|---|---|
| LNCRNA1 | AC104561.4 | 1 |
| LNCRNA2 | AP000224.1 | 2 |
| LNCRNA3 | AC098484.1 | 3 |
| LNCRNA4 | AC124283.1 | 4 |

As used herein, the term "prognosis" refers to a relative probability that a certain future outcome may occur in a patient. For example, in the context of the present disclosure, prognosis can refer to the likely occurrence of post-Chikungunya Chronic Inflammatory Joint Disease or its severity. The terms are not intended to be absolute, as will be appreciated by any one of skill in the field of medical diagnostics.

In a preferred embodiment, a "poor prognosis" in the context of the present disclosure means that a patient is at higher risk of developing post-Chikungunya Chronic Inflammatory Joint Disease.

As used herein, the wording "predicting the risk of developing Post-Chikungunya Chronic Inflammatory Joint Disease" means assigning an increased or decreased probability of having/developing Post-Chikungunya Chronic Inflammatory Joint Disease and/or assigning an increased or decreased probability of having a poor prognosis for a subject having developed Post-Chikungunya Chronic Inflammatory Joint Disease, as compared to the average risk in a population. Of course, an increased probability does not mean that the subject will develop Post-Chikungunya Chronic Inflammatory Joint Disease or will have a poor prognosis for Post-Chikungunya Chronic Inflammatory Joint Disease. The method may not also give a precise probability for such risk but may give a relative risk assessment as compared to the average risk in a given population.

### Providing a biological sample

The method of the invention is an *in vitro* method which can be carried out on any appropriate biological sample obtained from said subject.

As used herein, the term "biological sample" refers to a sample that contains nucleic acid materials reflecting the genomic information of cells, tissue or organs of the subject.

For example, said biological sample may be obtained from urine, blood including without limitation peripheral blood or plasma or serum, stool, sputum, saliva, bronchoalveolar fluid, endotracheal aspirates, wounds, cerebrospinal fluid, lymph node, exudate and more generally any human biopsy tissue or body fluids, tissues or materials. In a preferred embodiment, the biological sample is a whole blood sample.

The method may further comprise a step of extracting nucleic acids, in particular the RNA, from the biological sample, wherein the presence of long non-coding RNA is detected in the extracted nucleic acids.

In specific embodiments, the biological sample has been obtained from a symptomatic subject with CHIKV infection, preferably from a subject within the acute or subacute phase of CHIKV infection. For example, the biological sample has been obtained from a symptomatic subject with CHIKV infection within 1 to 90 days, within 1 to 60 days or within 1 to 30 days following the start of the first symptoms of chikungunya disease. Advantageously, the biological sample has been obtained from a symptomatic subject with CHIKV infection within 1 to 30 days following the first symptoms of chikungunya disease.

It is well known that total RNA of individuals can be easily extracted and purified from individual blood sample or saliva. Therefore, in a preferred embodiment, said biological sample is whole blood sample or a derivative thereof such as plasma or serum. More specifically, in preferred embodiments, said total RNA can be extracted and purified from whole blood sample using a composition including a reagent for RNA stabilization such as Tempus^{®} tube or PAXgene^{®} tube (Qiagen) (which reagent for RNA stabilization is also described in US Patents 6,602,718 and 6,617,170, herein incorporated by reference) or by using the MagMax^{™} for Stabilized Blood Tubes RNA Isolation Kit (ThermoFisher) according to the manufacturer's instructions, or by using the MagMax^{™} for Stabilized Blood Tubes RNA Isolation Kit (ThermoFisher) according to the manufacturer's instructions.

### The pCHIKV-CIJD Biomarkers

As used herein, the term "biomarker" is an objectively measurable biological characteristic which represents an indicator of normal or pathological biological processes or of pharmacological response to a therapeutic intervention. In the context of the present disclosure, the biomarkers refer to certain long non-coding RNA identified herein, of which the expression level is associated with an increased or decreased risk of developing post Chikungunya Chronic Inflammatory Joint Disease.

The inventors have indeed identified that the expression levels of certain long non-coding RNA (lnc-RNA) are associated to increased or decreased risk of developing post Chikungunya Chronic Inflammatory Joint Disease in a subject. Such newly identified biomarker are referred herein as pCHIKV-CIJD Biomarkers.

As used herein, a "long non coding RNA" is a type of RNA, generally defined as transcripts more than 200 nucleotides that are not translated into protein. This arbitrary limit distinguishes IncRNAs from small non-coding RNAs, such as microRNAs (miRNAs), Piwi-interacting RNAs (piRNAs), small nucleolar RNAs (snoRNAs) and other short RNAs. LncRNA includes long intervening/intergenic RNAs (lincRNAs) which are sequences of IncRNAs which do not overlap protein-coding genes.

The pCHIKV-CIJD Biomarkers for use in the methods and kits of the present disclosure, include any lnc-RNA sequences as described in the following Table 1 or a variant sequence thereof having at least 95% identity, and more preferably at least 96%, 97%, 98% or at least 99% identity with any of the sequences identified in SEQ ID NOs 1-4 or their corresponding cDNA sequences of SEQ ID NOs 5-8.

**Table 1**

| Lnc-RNA# | Reference | SEQ ID NO: |
|---|---|---|
| LNCRNA1 | AC104561.4 | 1 |
| LNCRNA2 | AP000224.1 | 2 |
| LNCRNA3 | AC098484.1 | 3 |
| LNCRNA4 | AC124283.1 | 4 |

Variations may correspond to natural genetic polymorphisms or transcription errors in the subject to be tested.

The "percentage of identity" as used herein may be determined between two nucleotide sequences after optimal sequence alignment. Optimal alignment of the sequences may be carried out but using for example algorithms of Smith and Waterman (J. Theor. Biol. 91(2):370-380, 1981), the algorithm of Needleman and Wunsch (J. Mol. Biol 48 (3): 443-453, 1972) or the method of Pearson and Lipman (PNAS USA 85(5): 2444-2448, 1988). Some softwares allow to implement some of these algorithms such as the Clustal W: improving the sensitivity of progressive multiple sequence alignment through sequence weighting, position-specific gap penalties and weight matrix choice). The best alignment (i.e. the one allowing to obtain the highest percentage of identity on the compared window), among those generated by these various methods is select. Typically, BLASTN program may be used as defaults a word length (W) of 11, an expectation (E) of 10, M=5, N=4, and a comparison of both strands.

### Detecting or evaluating the expression of one or more of the pCHIKV-CIJD Biomarkers

The method of the disclosure comprises a step of detecting and/or evaluating the expression of one or more of the pCHIKV-CIJD biomarkers in a biological sample.

As used herein, the term "detecting the expression of a pCHIKV-CIJD biomarker" means the demonstration of the expression of said biomarker, without necessarily a quantitative measurement. In the case of a transcript, in particular an mRNA transcript, the detection can be carried out by a direct method, by any method known to one skilled in the art making it possible to determine the presence of said transcript in a sample, or by indirect detection of the transcript after their transformation into DNA, or after amplification of said transcript or after amplification of the DNA obtained after transformation of said transcript into DNA.

As used herein, the term "evaluating" typically include the steps of (a) quantifying the expression of one or more of the selected pCHIKV-CIJD biomarkers in a biological sample obtained from said subject to obtain expression values, and (b) comparing the obtained expression value of one or more pCHIKV-CIJD biomarkers to corresponding control values, wherein differences, in particular an increase, in the expression values compared to the respective control values is indicative that the subject is at risk of developing pCHKV-CIJD.

The term "quantifying the expression" refers to quantitatively evaluating the expression level of the long non-coding RNA (pCHIKV-CIJD biomarker) in the biological sample.

Expression of the biomarkers can be advantageously quantified by determining RNA expression level of the long non-coding RNA (pCHIKV-CIJD biomarker) in the biological sample of a subject, for example a blood sample. The quantification may be relative (by comparing the amount of a pCHIKV-CIJD biomarker to known average amount of said respective pCHIKV-CIJD biomarker in a control population, for example and detecting "higher" or "lower" amount compared to that control) or more precise, at least to determine the specific amount relative to a known control amount. In specific embodiments, said control amount is the average expression of said pCHIKV-CIJD biomarker in a population which has not developed pCHIKV-CIJD.

In specific embodiments, a subject is considered at risk if said expression of said pCHIKV-CIJD (typically from a whole blood sample) is higher, preferably at least twice higher than the expression in a control population which has not developed pCHIKV-CIJD.

Such quantification methods may alternatively include detection and quantification of the corresponding gene expression level of said pCHIKV-CIJD biomarker which encompasses the quantification of corresponding mRNA of said pCHIKV-CIJD biomarker, by any means known to the art.

In any cases, a general principle of such detection and quantification assays involves preparing a sample or reaction mixture that may contain a pCHIKV-CIJD biomarker (or their amplified corresponding nucleic acids, and a probe under appropriate conditions and for a time sufficient to allow the pCHIKV-CIJD biomarker (or their amplified corresponding nucleic acids) and probe to interact and bind, thus forming a complex that can be detected (and quantified) in the reaction mixture.

The detection and/or quantification methods of a transcript RNA can be conducted in a variety of ways. Examples of RNA quantification method which may be used in the methods of the present disclosure include without limitation, hybridization methods, in situ hybridization, Northern Blot, amplification methods or sequencing methods (such as high throughput sequencing), in particular quantitative polymerase chain reaction such as Real-Time quantitative PCR (RT-qPCR). Appropriate conditions to the particular assay and components thereof will be readily determined by the one skilled in the art.

In one specific embodiment, the expression of said one or more pCHIKV-CIJD Biomarker is determined from total RNA extracted from the biological sample, typically by RT-qPCR using primers specific for amplifying one or more of SEQ ID NOs 1-8.

In specific methods using quantitative PCR, the amplified nucleic acid fragment may be a fragment of at least 20, 30, 40, 50, 100, 200, 300 or at least 500 consecutive nucleotides, for example comprised between 20 and 1000 consecutive nucleotides, preferably between 30 and 200 consecutive nucleotides. The one man skilled in the art will adapt the size of the fragments according to the method used for each pCHIKV-CIJD biomarker.

Thus, in a number of the quantification methods, primers and probes which span one or more fragments of the pCHIKV-CIJD Biomarkers (e.g. one or more fragments of any of SEQ ID NOs 1-8 as described in the above paragraphs) may be used to detect and/or evaluating the expression of said pCHIKV-CIJD Biomarker.

As used herein, the term "probe" or "primer" refers to one or more synthetic nucleic acid fragments whose specific hybridization to a sample can be detected. A probe or primer can be of any length depending on the particular technique it will be used for.

As used herein, the term "hybridization" refers to the process during which, under appropriate conditions, two nucleotide fragments, such as for example a hybridization probe and a target nucleotide fragment, or a primer and a strand of cDNA, are capable of forming a double strand with stable and specific hydrogen bonds. Hybridization conditions are determined by the stringency, i.e. the stringency of the operating conditions. The stringency of the conditions under which a hybridization reaction is to be performed will depend primarily on the used hybridization probes. In general, depending on the length of the used hybridization probes, the temperature for the hybridization reaction is comprised between about 20 and 70°C, particularly 35 and 65°C, in saline solution at a concentration of about 0.5 to 1M.

Such probes or primers which may be used in the methods of the invention may typically be short nucleic acid molecules, for instance DNA oligonucleotides of 10 nucleotides or more in length, which can be annealed to the complementary target nucleic acid molecule by nucleic acid hybridization to form a hybrid between the primer or probe and the target nucleic acid strand.

The probe or primers can be unlabelled or labelled so that its binding to a target sequence can be detected (e.g. with a FRET donor or acceptor label).

A primer can be extended along the target nucleic acid molecule by a polymerase enzyme.

Therefore, primers can be used to amplify the target nucleic acid molecule, such as fragments of any of the LNCRNA 1-4 described in the above Table 1, and/or their variant sequences.

The specificity of a probe or a primer increases with its length. Thus, for example, a probe or primer that includes 30 consecutive nucleotides will anneal to a target sequence with a higher specificity than a corresponding primer of only 15 nucleotides. Thus, to obtain greater specificity, probes and primers can be selected that include at least 15, 20, 25, 30, 35, 40, 45, 50, 55, 60, 65, 70 or more consecutive nucleotides.

In particular examples, a primer is at least 15 nucleotides in length, such as at least 15 contiguous nucleotides complementary to a target nucleic acid molecule. Particular lengths of primers that can be used to practice the methods of the present disclosure include primers having at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 45, at least 50, at least 55, at least 60, at least 65, at least 70, or more contiguous nucleotides complementary to the target nucleic acid molecule to be amplified, such as a primer of 15-70 nucleotides, 15-60 nucleotides, 15-50 nucleotides, or 15-30 nucleotides.

An "upstream" or "forward" primer is a primer 5' to a reference point on a nucleic acid sequence. A "downstream" or "reverse" primer is a primer 3' to a reference point on a nucleic acid sequence. In general, at least one forward and one reverse primer (the "pair of primers") are included in an amplification reaction.

Nucleic acid probes and primers, or pair of primers, can be readily prepared based on the nucleic acid sequence of SEQ ID NOs 1-8.

PCR primer pairs can be derived from a known sequence by using computer programs intended for that purpose such as Primer 3 (v. 0.4.0 Whitehead Institute for Biomedical Research, Steve Rozen, and Helen Skaletsky).

In other embodiments, the long non-coding RNA or their amplified products and transformed products (e.g. from RNA to DNA) are detected by specific hybridization of nucleic acid probes, such as oligonucleotide probes to genomic DNA or RNA transcripts or corresponding cDNA, potentially containing the pCHIKV-CIJD Biomarkers, for example containing any one of SEQ ID NOs 1-8.

Such probes can also be immobilized on a solid surface (such as nitrocellulose, glass, quartz, fused silica slide) as in an array or microarray or DNA chip. One of skill in the art will recognize that the precise sequence of particular probes and primers can be modified from the target sequence to a certain degree to produce probes that are "substantially identical" or "substantially complementary" to a target sequence, while retaining the ability to specifically bind to (i.e. hybridize specifically to) the same targets from which they are derived.

In the context of the present disclosure, the terms "capable of hybridizing to" and "binds specifically to", which are used interchangeably, refer to a polynucleotide sequence that forms Watson-Crick bonds with a complementary sequence. One of skill will understand that the percent complementary need not be 100% for hybridization or specific binding to occur, depending on the length of the polynucleotides, length of the complementary region and stringency of the conditions. For example, a primer or probe is at least 60%, 70%, 80%, 90%, 95%, 99% or 100% complementary over the stretch of the complementary region.

In one specific embodiment, the method comprises the steps of:
(a) Obtaining a whole blood sample from a patient having chikungunya fever,
(b) Contacting the whole blood sample with reagents specific to products of expression of 1 to 4 target lnc-RNA selected from the group consisting of the long non-coding RNA having the sequence of SEQ ID NOs 1 to 4, and
(c) Measuring the expression level of said target lnc-RNA in the whole blood sample.

The specific reagents can be amplification primers or hybridization probes as defined above.

### Comparing expression level of the pCHIKV-CIJD biomarkers to a control value

In specific embodiment of the method of the disclosure, the quantifying step thus allows to obtain an "expression value" for each biomarker tested in the biological sample, for use in the comparing step.

For ease of use in the comparing step, said expression value may consist of a normalized (relative) value which is obtained after comparison of the absolute expression level value with a reference value, said reference value consisting for example of the expression level value of a reference RNA transcript in the biological sample.

Each expression level value obtained after quantification of the expression of one or more of the pCHIKV-CIJD biomarkers in a subject, may then be compared with a corresponding control value, allowing to determine whether the subject is at risk or not of developing pCHIKV-CIJD.

Preferably, said expression level value consists of a normalized relative value which is obtained by the ratio of the absolute expression level value with a reference value, said reference value consisting for example of the expression level value of constitutive (or reference) RNA, such as housekeeping genes GAPDH, β-actin, 18S RNA or peptidylprolyl isomerase A (PPIA), preferably the housekeeping gene GAPDH.

Said normalized relative value may be compared with a normalized control value, for example, the mean value of normalized (relative) mean value of symptomatic CHIKV subjects which have not developed pCHIKV-CIJD.

Said control value can also be determined by routine experimentation depending on the quantification methods and the CIJD biomarkers that will be used for the methods of the disclosure.

For example, said control value corresponds to the average expression level value measured for a control group of symptomatic CHIKV subjects which have not developed pCHIKV-CIJD, and a patient is predicted to be at risk of developing pCHIKV-CIJD if the corresponding expression value of one or more of pCHIKV-CIJD Biomarker is statistically higher, than the corresponding control value, for example twice higher as compared to the corresponding control value, at least in one of the pCHIKV-CIJD Biomarker, preferably for the pCHIKV-CIJD Biomarker of SEQ ID NO:1.

By "statistically higher", the expression value is at least 10%, 20%, 30%, 40%, 50%,60%, 70%, 80%, 90%. It is also meant that the mean expression value is at least twice higher than the control value and the p-value is less than 0.05.

Alternatively, said control value corresponds to the average expression level value measured for patients which exhibit pCHIKV-CIJD, and a patient is predicted to be at risk of developing pCHIKV-CIJD when the expression level value is statistically different from the control value, and in particular higher from the control value.

The comparison step of the methods of the disclosure may be carried out manually or computer assisted. For a computer-assisted comparison, the expression values may be compared to control values which are stored in a database by a computer program. The computer program may further evaluate the result of the comparison, i.e. automatically provide the desired assessment in a suitable output format.

As it is shown in the examples, each of the pCHIKV-CIJD biomarkers according to the Table 1 is relevant for predicting a risk of developing pCHIKV-CIJD. Statistical relevance may be improved by combining the pCHIKV-CIJD biomarkers in the assays. In specific embodiments, the expression level of 2, 3 or 4 of the selected biomarkers as defined in Table 1 is evaluated.

In specific embodiments, the methods of the present disclosure comprise at least evaluating the expression of the long non-coding RNA of SEQ ID NO:1 (LNCRNA1), optionally in combination with one or more additional biomarkers. For example, in such specific embodiment, a higher expression of LNCRNA1 as compared to corresponding expression value measured in symptomatic patients which do not develop pCHIKV-CIJD symptoms, is predictive of a risk of developing pCHIKV-CIJD, preferably with a sensitivity higher than 70% and a specificity higher than 90%.

In specific embodiments, the methods of the present disclosure comprise at least evaluating the expression of the long non-coding RNA SEQ ID NO:1 (LNCRNA1) and the long non-coding RNA of SEQ ID NO:2 (LNCRNA2). In such specific embodiments, a higher expression of both LNCRNA1 and LNCRNA2 as compared to corresponding expression values measured in symptomatic patients which do not develop pCHIKV-CIJD symptoms, is predictive of a risk of developing pCHIKV-CIJD, preferably with a sensitivity higher than 70% and a specificity higher than 90%.

In specific embodiments, the methods of the present disclosure comprise at least evaluating the expression of (i) the long non-coding RNA of SEQ ID NO:1 (LNCRNA1); (ii) the long non-coding RNA of SEQ ID NO:2 (LNCRNA2) and (iii) the long non-coding RNA of SEQ ID NO:3 (LNCRNA3). In such specific embodiments, a higher expression of the three selected biomarkers as compared to corresponding expression value measured in symptomatic patients which do not develop pCHIKV-CIJD symptoms, is predictive of a risk of developing pCHIKV-CIJD, preferably with a sensitivity and specificity higher than 95%.

In specific embodiments, the methods of the present disclosure comprises at least evaluating the expression of (i) the long non-coding RNA of SEQ ID NO:1 (LNCRNA1); (ii) the long non-coding RNA of SEQ ID NO:2 (LNCRNA2) and (iii) the long non-coding RNA of SEQ ID NO:4 (LNCRNA4). In such specific embodiments, a higher expression of the three selected biomarkers as compared to corresponding expression value measured in symptomatic patients which do not develop pCHIKV-CIJD symptoms, is predictive of a risk of developing pCHIKV-CIJD, preferably with a sensitivity and specificity higher than 95%.

In specific embodiments, the methods of the present disclosure comprise at least evaluating the expression of (i) the long non-coding RNA of SEQ ID NO:1 (LNCRNA1); (ii) the long non-coding RNA of SEQ ID NO:2 (LNCRNA2); (iii) the long non-coding RNA of SEQ ID NO:3 (LNCRNA3) and (iv) the long non-coding RNA of SEQ ID NO:4 (LNCRNA4).

The comparing step may not necessarily include a separate comparison of the expression values of each biomarker with their corresponding control values. In specific embodiments, a multi-biomarker score value can be obtained by combining together the expression values or their normalized values and compared to a corresponding multibiomarker score control value.

### Patient stratification methods

The invention further relates to patient stratification methods based on the risk assessment provided by the above-described methods.

In particular, a patient identified at increased risk of developing pCHIKV-CIJD may have adapted therapeutic or prophylactic treatments in order to reduce the risk of developing pCHIKV-CIJD.

Accordingly, the disclosure further includes a method comprising:
(i) identifying whether a subject is at increased risk of developing pCHIKV-CIJD according to the above defined methods, and,
(ii) administering a suitable therapeutic or prophylactic treatment of pCHIKV-CIJD in said subject presenting a risk of developing pCHIKV-CIJD.

As used herein, the term "therapeutic or prophylactic treatment" refers to any measures, wherein the object is to prevent or slow down (lessen) the targeted pathologic condition or disorder or slow down or relieve one or more of the symptoms of the disorder.

Examples of suitable therapeutic or prophylactic treatment may include without limitation treatment with anti-inflammatory drugs, for example for treating rheumatoid conditions, such as methotrexate.

In specific embodiments, said therapeutic or prophylactic treatment is advantageously administered within 3 months, 2 months or 1 month after the start of the first symptoms of Chikungunya disease, and preferably within the acute phase of Chikungunya disease in said subject identified at risk of developing pCHIKV-CIJD.

### Kits for performing the method

Kits may be prepared for performing the above-described methods. In particular, one object of the disclosure relates to a kit comprising means for amplifying and/or detecting at least one sequence selected from the long non-coding RNA sequences identified in SEQ ID NO: 1-4, wherein the amplification and/or detection means allow the amplification, the detection and/or the quantification, of 1, 2, 3 or all 4 long non-coding RNA sequences in a biological sample, and, optionally instructions for use of the kit.

In specific embodiments,
(i) the amplification means comprises at least one pair of primers for specific amplification of one of the selected biomarkers sequences of SEQ ID NOs 1-4 or variant biomarker sequences having at least 95% identity to any of SEQ ID NOs 1-4, each amplification primer comprising or consisting of a nucleotide sequence identical or complementary to a part of any of SEQ ID NOs 1-8, or to a variant sequence having at least 95% identity to any of SEQ ID NOs 1-8; or
(ii) the detection means comprises at least a nucleic acid probe whose nucleotide sequence comprises or consists of a complementary sequence to at least part of any of SEQ ID NOs 1-8 or to a variant sequence having at least 95% identity to any of SEQ ID NOs 1-8.

In particular, the kit of the disclosure may comprise probes that binds specifically to any one of SEQ ID NOs 1-8.

In other embodiments, the kit of the disclosure may comprise primers that are capable of amplifying a long non-coding RNA from a biological sample of a subject comprising any one of SEQ ID NOs 1-4 or a fragment thereof.

For example, the kit can include primers for amplifying at least LNCRNA1 as described above, or a variant sequence having at least 95% identity to SEQ ID NO:1 or SEQ ID NO:5, typically by quantitative PCR or quantitative RT-PCR.

In other specific embodiments, the kit can include primers for amplifying at least both biomarkers LNCRNA1 and LNRNA2, or their variant sequences having at least 95% identity to SEQ ID NO:1 or SEQ ID NO:5 and SEQ ID NO:2 or SEQ ID NO:6 respectively, typically by quantitative PCR or quantitative RT-PCR.

In other specific embodiments, the kit can include primers for amplifying at least the three biomarkers LCNRNA1, LNCRNA2 and LNCRNA3, or their variant sequences having at least 95% identity to SEQ ID NO: 1 or SEQ ID NO:5, SEQ ID NO:2 or SEQ ID NO:6 and SEQ ID NO:3 or SEQ ID NO:7 respectively, typically by quantitative PCR or quantitative RT-PCR.

In other specific embodiments, the kit can include primers for amplifying at least the three biomarkers LCNRNA1, LNCRNA2 and LNCRNA4, or their variant sequences having at least 95% identity to SEQ ID NO: 1 or SEQ ID NO:5, SEQ ID NO:2 or SEQ ID NO:6 and SEQ ID NO:4 or SEQ ID NO:8, respectively, typically by quantitative PCR or quantitative RT-PCR.

The kit can further include one or more of a buffer solution, a conjugating solution for developing the signal of interest, or a detection reagent for detecting the signal of interest, each in separate packaging, such as a container.

In another example, the kit includes a plurality of size-associated marker target nucleic acid sequences for hybridization with a detection array. The target nucleic acid sequences can include oligonucleotides such as DNA, RNA, and peptide-nucleic acid, or can include PCR fragments.

The kit can also include instructions in a tangible form, such as written instructions or in a computer-readable format.

### Specific Embodiments

The disclosure further relates, without limitation, to the following embodiments E1-E27:
E1: An *in vitro* method for predicting the risk of developing Post-Chikungunya Chronic Inflammatory Joint Disease (pCHIKV-CIJD) in a subject, said method comprising detecting and/or evaluating the expression of one or more biomarkers in a biological sample obtained from said subject, for example a blood sample, wherein said one or more biomarkers are selected from the following long non-coding RNA sequences identified below as SEQ ID NOs 1-4 or a variant sequence thereof having at least 95% identity with one of the sequences identified in SEQ ID NOs 1-4:

| Lnc-RNA# | Reference | SEQ ID NO: |
|---|---|---|
| LNCRNA1 | AC104561.4 | 1 |
| LNCRNA2 | AP000224.1 | 2 |
| LNCRNA3 | AC098484.1 | 3 |
| LNCRNA4 | AC124283.1 | 4 |

E2. The method of E1, wherein the evaluating step includes (a) quantifying the expression of said one or more selected biomarkers in a biological sample obtained from said patient to obtain an expression value for one or more of said biomarkers, and (b) comparing each expression value obtained at step (a) to a control value and (c) determining the risk of developing pCHIKV-CIJD based on said comparison.
E3. The method of E1 or E2, wherein said control value corresponds to average expression value of said biomarker as measured in symptomatic patients which do not develop pCHIKV-CIJD.
E4. The method of any one of E1-E3 wherein the expression of 2, 3 or 4 of the selected biomarkers is quantified.
E5. The method of any one of E1-E4, wherein a higher expression value of said one or more selected biomarker as compared to corresponding control value measured in control group of patients which do not develop pCHIKV-CIJD is predictive of a risk of developing pCHIKV-CIJD.
E6. The method of any one of E1-E5, which comprises at least evaluating the expression of the long non-coding RNA of SEQ ID NO: 1 (LNCRNA1).
E7. The method of E6, wherein a higher expression of LNCRNA1 as compared to corresponding expression value measured in symptomatic patients which do not develop pCHIKV-CIJD, is predictive of a risk of developing pCHIKV-CIJD, preferably with a sensitivity higher than 70% and a specificity higher than 90%.
E8. The method of E6, further comprising evaluating the expression of the long non-coding RNA of SEQ ID NO:2 (LNCRNA2).
E9. The method of E8, wherein a higher expression of both LNCRNA1 and LNCRNA2 as compared to corresponding expression values observed in symptomatic patients which do not develop pCHIKV-CIJD symptoms, is predictive of a risk of developing pCHIKV-CIJD, preferably with a sensitivity higher than 70% and a specificity higher than 90%.
E10. The method of E6, further comprising evaluating the expression of the long non-coding RNA of SEQ ID NO:2 (LNCRNA2) and the long non-coding RNA of SEQ ID NO:3 (LNCRNA3).
E11. The method of E6, further comprising evaluating the expression of the long non-coding RNA of SEQ ID NO:2 (LNCRNA2) and the long non-coding RNA of SEQ ID NO:4 (LNCRNA4).
E12. The method of E10 or E11, wherein a higher expression of the three selected biomarkers as compared to corresponding expression value observed in symptomatic patients which do not develop pCHIKV-CIJD symptoms, is predictive of a risk of developing pCHIKV-CIJD, preferably with a sensitivity and specificity higher than 95%.
E13. The method according to any one of E1 to E12, wherein the expression of said one or more selected biomarkers is determined by a RNA quantification method, for example selected from the group consisting of hybridization methods, in situ hybridization, Northern blot, amplification methods or sequencing methods, in particular quantitative PCR such as RT-qPCR.
E14. The method according to any one of E1 to E13, further comprising extracting total RNA from the biological sample, wherein the expression of said one or more selected biomarkers is determined in the extracted total RNA.
E15. The method according to any one of E1 to E14, wherein the biological sample has been obtained from a subject within the acute or subacute phase of CHIKV infection, preferably within 2 to 30 days following the start of the first symptoms of chikungunya disease.
E16. A kit comprising means for amplifying and/or detecting at least one sequence selected from the long non-coding RNA sequences identified in SEQ ID NOs: 1-4, wherein the amplification and/or detection means allow the amplification, the detection and/or the quantification, of 1, 2, 3 or all 4 long non-coding RNA sequences in a biological sample.
E17 . The kit of E16, wherein
(i) the amplification means comprises at least one pair of primers for specific amplification of one of the selected biomarkers sequences of SEQ ID NOs 1-4 or variant biomarker sequences having at least 95% identity to any of SEQ ID NOs: 1-4, each amplification primer comprising or consisting of a nucleotide sequence identical or complementary to a part of any of SEQ ID NOs: 1-8, or to a variant sequence having at least 95% identity to any of SEQ ID NOs: 1-8; or
(ii) the detection means comprises at least a nucleic acid probe whose nucleotide sequence comprises or consists of a complementary sequence to at least part of any of SEQ ID NOs: 1-8, or to a variant sequence having at least 95% identity to any of SEQ ID NOs:1-8.
E18. The kit of E16 or E17, comprising at least a pair of primers for specifically amplifying the biomarker LNCRNA1 of SEQ ID NO:1 or a variant sequence having at least 95% identity to SEQ ID NO:1 or SEQ ID NO:5, typically by quantitative PCR or quantitative RT-PCR.
E19. The kit of any one of E16-E18, further comprising at least a pair of primers for specifically amplifying the biomarker LNCRNA2 of SEQ ID NO:2 or a variant sequence having at least 95% identity to SEQ ID NO:2 or SEQ ID NO:6, typically by quantitative PCR or quantitative RT-PCR.
E20. The kit of any one of E16-E19, further comprising at least a pair of primers for specifically amplifying the biomarker LNCRNA3 of SEQ ID NO:3 or a variant sequence having at least 95% identity to SEQ ID NO:3 or SEQ ID NO:7, typically by quantitative PCR or quantitative RT-PCR.
E21. The kit of any one of E16-E29, further comprising at least a pair of primers for specifically amplifying the biomarker LNCRNA4 of SEQ ID NO:4 or a variant sequence having at least 95% identity to SEQ ID NO:4 or SEQ ID NO:8, typically by quantitative PCR or quantitative RT-PCR.
E22. The kit of E16 or E17, comprising at least a nucleic acid probe for specifically detecting the biomarker LNCRNA1 of SEQ ID NO:1 or a variant sequence having at least 95% identity to SEQ ID NO:1 or SEQ ID NO:5, typically by quantitative detection.
E23. The kit of E16, E17 or E22, comprising at least a nucleic acid probe for specifically detecting the biomarker LNCRNA2 of SEQ ID NO:2 or a variant sequence having at least 95% identity to SEQ ID NO:2 or SEQ ID NO:6, typically by quantitative detection.
E24. The kit of E16, E17, E22 or E23, comprising a nucleic acid probe for specifically detecting the biomarker LNCRNA3 of SEQ ID NO:3 or a variant sequence having at least 95% identity to SEQ ID NO:3 or SEQ ID NO:7, typically by quantitative detection.
E25. The kit of E16, E17, E22 or E23, comprising at least a nucleic acid probe for specifically detecting the biomarker LNCRNA4 of SEQ ID NO:4 or a variant sequence having at least 95% identity to SEQ ID NO:4 or SEQ ID NO:8, typically by quantitative detection.
E26. The kit of any of E16-E25, for predicting the risk of developing Chronic Inflammatory Joint Disease Post-Chikungunya (pCHIKV-CIJD) in a subject.
E27. A method of treating or preventing Chronic Inflammatory Joint Disease Post-Chikungunya (pCHIKV-CIJD) in a subject in need thereof, said method comprising determining the risk of developing pCHIKV-CIJD in a subject by a method according to any of E1-E15, and administering a suitable therapeutic or prophylactic treatment of pCHIKV-CIJD in said subject selected as presenting a risk of developing pCHIKV-CIJD.
E28. The method of E27, wherein said suitable therapeutic or prophylactic treatment of pCHIKV-CIJD includes anti-inflammatory treatment, for example selected from antirheumatic drugs, such as methotrexate.
E29. The method of E27 or E28, wherein said suitable treatment is administered prior to 3, 2 or 1 months after the start of the first symptoms of chikungunya disease.

The invention will now be further illustrated by the following figures and examples. However, these examples and figures should not be interpreted in any way as limiting the scope of the present invention.

### BRIEF DESCRIPTION OF THE FIGURES

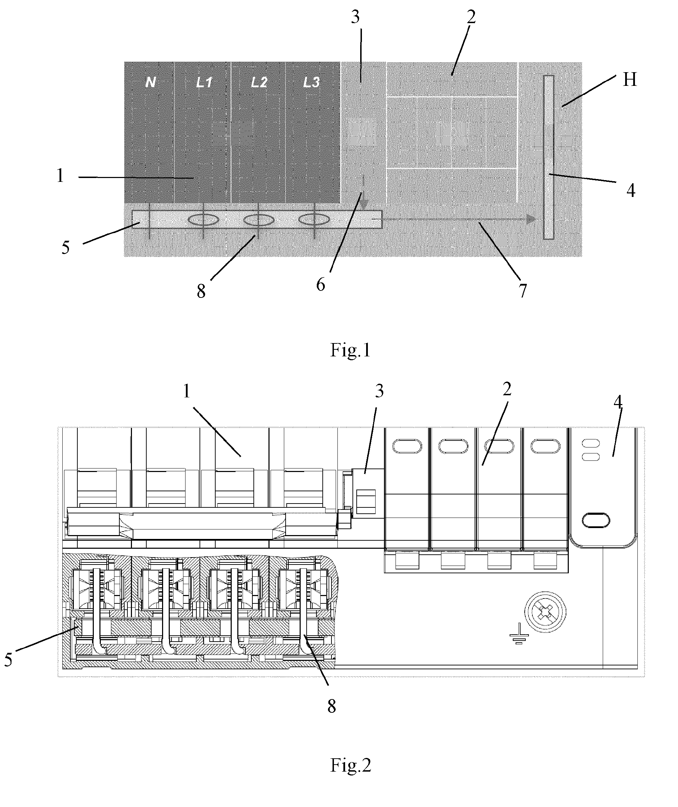

**Figure 1****:** Representative ROC curve considering the expression values of lnc-RNA (A) AC104561.4 and (B) the combination of AC104561.4 and AP000224.1; and violin plot including the distribution of expression values of (C) AC104561.4 and (D) AP000224.1, among pCHIKV-CIJD (right plot) samples and control samples (left plot).

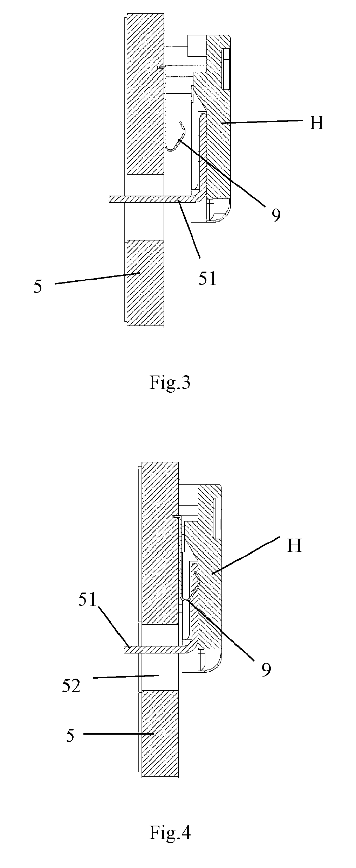

**Figure 2****:** Representative ROC curve considering the expression values of (A) the combination of AC104561.4, AP000224.1 and AC098484.1 and (B) the combination of AC104561.4, AP000224.1 and AC124283.1; and violin plot including the distribution of expression values of (C) AC098484.1 and (D) AC124283.1, among pCHIKV-CIJD (right plot) samples and control samples (left plot).

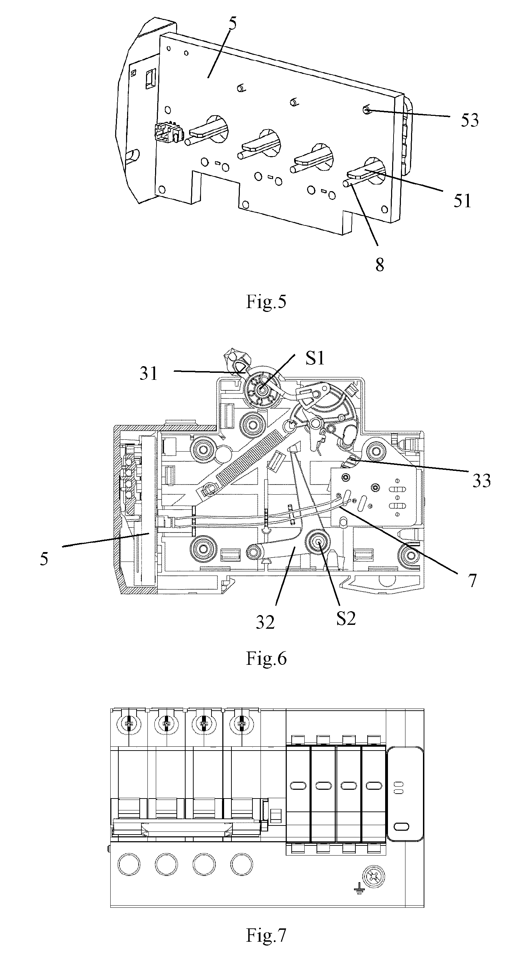

**Figure 3****:** Flowchart representing the cohort design.

### EXAMPLES

### Example 1: Identification of predictive biomarkers for developing post-Chikungunya Chronic Inflammatory Disease

### Materials and methods

### Cohort design

A prospective longitudinal cohort at the Evandro Chagas National Institute of Infectology - INI / FIOCRUZ - Rio de Janeiro-RJ was established between April and August 2019, including patients who were suspected to be infected by CHIKV.

The cohorts included 186 patients with the classic symptoms of arbovirus disease, such as fever, headache, and joint pain, of which 169 were confirmed by molecular tests and serology to be positive for CHIKV. Indeed, RT-PCR detected viral RNA in 137 patients and the ELISA test found IgM in 32 more patients, totalizing 169 patients in the CHIKV sub-cohort confirmed by laboratory examination.

These positive-CHIKV patients had two follow-up visits at 21 days (D21) and 90 days (D90) after inclusion (D0). During these follow-up visits, new collections of biological material and clinical information about the pain of joint symptoms were carried out. 131 patients returned for the D21 visit, 112 of whom were still symptomatic at the time and 86 patients returned for the D90 follow-up visit, 56 of whom still had symptoms.

Those patients who, at D90, remained with joint symptoms and signs of arthritis (n=39), were referred for ultrasound imaging examination and 29 patients showed image alteration and have been classified as patient having pCHIKV-CIJD.

Lastly, 33 patients from the cohort who did not present pCHIKV-CIJD were selected as a comparison group. This pairment was made considering, as a criteria, that there were no significant differences regarding the age (pCHIKV-CIJD age median = 49.5/non-pCHIKV-CIJD age median = 43; p = 0.31) and sex (pCHIKV-CIJD = 25 female and 4 male/non-pCHIKV-CIJD = 22 female and 11 male; p = 0.08) of these patients that could imply a bias in the subsequent analyses.

### RNA sequencing

After establishing the study groups, we performed sequencing experiments to select the potential early predictive biomarkers of pCHIKV-CIJD.

First, during the inclusion visit to the care unit and, thus, in the acute phase of the disease, 3mL of whole blood was collected from the patients in the Tempus^{™} Tube (ThermoFisher, Waltham, MA, United States), which are recommended for stabilization and isolation of total RNA from whole blood for gene expression analysis.

This special tube contains 6mL of stabilizing reagent and when the patient's blood is mixed with this reagent, lysis occurs, cellular RNases are inactivated, and the RNA precipitated selectively. Furthermore, the genomic DNA and proteins remain in solution.

After collecting, the material was kept at -80 °C. The materials were available from 23 of 29 patients from the pCHIKV-CIJD group and 25 of 33 patients from the non-pCHIKV-CIJD group. However, after reviewing the clinical data, four patients classified as non-pCHIKV-CIJD group showed clinical signs of arthritis. Although the alterations were not confirmed by ultrasound examination, these patients have been excluded to reduce the possible bias that could occur due to a misclassification. Finally, 44 samples were available for RNA extraction and sequencing analysis.

To obtain total RNA from the whole blood collected in the Tempus tube, we used the MagMax^{™} for Stabilized Blood Tubes RNA Isolation Kit (ThermoFisher) according to the manufacturer's instructions.

Briefly, the total content (9 ml) of the tube was diluted in Tempus^{™} PBS in a 50 ml conical tube and centrifuged for RNA precipitation. The supernatant was discarded, and the pellet washed with Pre-Digestion Wash Buffer. The pellet holding the RNA was resuspended in a solution containing protease and the total content was transferred to a 1.5mL tube. Then, treatment with DNase, precipitation with 100% isopropanol and purification using magnetic beads was performed. Elution of total RNA was performed in 40 µL of Elution Buffer. The purity of the material was verified by spectrophotometry using Nanodrop^{™} 2000 (ThermoFisher) and the quantification and integrity check of the material was measured in the Agilent Bioanalyzer 2100 using Agilent RNA 6000 Pico Kit (Agilent Technologies, Santa Clara, CA, USA). All samples had more than 70% of the RNA fragments larger than 200bp (DV200 > 70%) and were considered of high quality, being considered suitable for the construction of the total RNA library using the TruSeq Stranded Total RNA Library Prep kit with RiboZero Gold (Illumina, San Diego, California, USA).

A total input of 500 ng of total RNA was used to prepare libraries by the ribosomal RNA depletion strategy, as recommended by the manufacturer. Finally, 2 pmol of the pool, with 12 libraries were applied onto NextSeq 500/550 High Output Kit v2.5 (150 Cycles) and sequenced using NextSeq 550 System (Illumina).

### Human transcriptome analysis

Gene count matrices were generated with the featureCounts function from the Rsubread package (v2.4.3) [Liao et al., 2019: FeatureCounts: an efficient general purpose program for assigning sequence reads to genomic features, Bioinformatics, Volume 30, Issue 7, 1 April 2014, Pages 923-930] in the R programming language (v4.0.5). The identification of differentially expressed genes was performed using the edgeR package (v3.32.1)

[McCarthy et al. 2012: Differential expression analysis of multifactor RNA-Seq experiments with respect to biological variation, Nucleic Acids Research, 40(10):4288-4297, 2012] through comparison pCHIKV-CIJD vs. non-pCHIKV-CIJD.

The comparison between disease conditions (pCHIKV-CIJD vs. non-pCHIKV-CIJD) at initial stage showed 28 up-regulated genes and 22 down-regulated genes in pCHIKV-CIJD strains, including two immunoglobulin gene, 14 non-coding RNA, 12 pseudogenes and 21 protein-coding gene.

### Statistics

To assess whether the difference in gene expression is able to distinguish the two study groups with high accuracy, we performed the ROC curve through the Epi package for R Studio version 1.2.5033, using the expression data for all 50 differentially expressed genes, individually or in combination.

### Results

After bioinformatics analysis, the total RNA sequencing of the samples has revealed that 50 differentially expressed genes (DEGs), 28 up-regulated and 22 down-regulated genes, were found in the pCHIK-CIJD group compared to the non-pCHIKV-CIJD group. Considering the list of DEGs obtained in the transcriptome analyses, the ROC curve analysis was performed to assess the ability of each of these genes, alone or in combination, to discriminate the group of patients with pCHIKV-CIJD.

First, each of the 50 genes was evaluated individually and the long-non-coding RNA (lnc-RNA) AC104561.4 showed good performance **(Sensitivity: 73.9%, Specificity: 90.5%, Accuracy: 84.3%,** **Figure 1A****).**

Aiming a better discrimination power, the ROC curve was performed using the combination of all genes in pairs or in triplets, totaling 1225 and 19600 different combinations, respectively. In this analysis, the combination in pairs that showed the best performance **(Sensitivity: 87%, Specificity: 100%, Accuracy: 96.9%,** **Figure 1B**) was the same AC104561.4 and AP000224.1, another lnc-RNA.

When separated into triplets, two combinations showed 100% accuracy **(Sensitivity: 100%, Specificity: 100%):** the combination of AC104561.4 and AP000224.1 with AC098484.1 **(****Figure 2A****)** and AC124283.1 **(****Figure 2B****),** all being up-regulated lnc-RNAs in the pCHIKV-CIJD group **(****Figures 1C, 1D****,** **2C and 2D****).**

Thus, four lnc-RNA showing high capacity to distinguish the group of patients with pCHIKV-CIJD, thus indicating that they are good biomarkers for detecting individuals who may develop this disease early in the acute phase of Chikungunya fever symptoms, enabling interventions in the clinical management that can assuage or even prevent the joint symptoms of these patients in the long term.

Importantly, although more than 85% of the human genome is transcribed, only less than 2% is transcribed into messenger RNA, that is, a protein-coding RNA. In this context, many non-coding RNAs are transcribed as functional RNAs of different sizes, structures, and biological functions. Those non-coding RNAs longer than 200 nucleotides are known as long-non-coding RNAs. These lnc-RNA participate in transcriptional and post-transcriptional regulation. In addition to their biological role, many studies show that lnc-RNAs can function as biomarkers for diagnosis and prognosis of various diseases. Compared to conventional biomarkers, lnc-RNA seems to have higher diagnostic and prognostic values, not only because of their tissue and disease specific expression patterns, but also due to their highly stable physical and chemical properties¹⁴.

These results may serve as a starting point for validations and identification of distinct molecules that could be exploited as biomarker candidates thereby helping in better patient management and public health.

### Example 2: Description of the long non-coding RNA for use as pCHIKV-CIJD biomarkers

| Lnc-RNA# | Reference | RNA SEQUENCE | SEQ ID NO: |
|---|---|---|---|
| LNCRNA1 | AC104561.4 | AGGACCGCCGAGCUUUGCUGAGGCAUCAUG GUGGGACUUAAAGAAGUAUCUUGGCCGGG CGCAGUGGCUCACACCUAUAAUCCCAGAAC UUUGGGAGGAUGAGGCAGGAGGAUCAAUU GAGUCACGGAGUUCAAGACCAGCCUGGGCA ACAGAGUGAGACUCUGUCUCUACAACAAAU UUUAAAAAAAUUAGUCAGGUAUGGUGACG UGCACGUAUAGUCUCAGCUACUUGGGAGGC UGAGGUGGGAGGAUCACUUGAGCUCGGGA GGUGGAGGUUGCAGUGAGCCAUGAUUGCG CCACUCACUCUAACAUAGGUGACAGAGUGA GAUCCCGUCUCCAAAAACAAACACAAACAA ACUAAAAGAAGUAUCAGCUGCUACACAUCU GGAAUGACAACAUAUGCAGAAUACAAAUG UAAAUAGAAAACAUAGAAUAUAAAUUAUG AGUUUUAGUUGAGUAACGCUGACUUGGAA UUGGAAGAACAUUUGCAGGCAUCAGUAAU GGGAGAGUAGAAAAUGUGAGAAAGAAACC UUAAGUCAAUUAAGAGAAUACACUCUGCA AAUUAGGGGUGGAUCCUUGGGUUAAUCUG CAGCCUGCAGUUGUACCCUGCUAGAAGCAG CUUCGAUGCCACUGAAGUUAAUCUUGGGUC CCAGUAAAUAUCUUCUCUCCUCUCUAUUAU CCACUGUGCAAGAUAUAUGUAAAUACUGCC AGAUACAUUUUUUAAAAGGCCCGGGUGCU AUCGUGUAUAGGUUGACAUUAAUGUACUC CCAGAAUGCCCAGGCAUGGUAUGGGGUGGC CACCCUAUGGUUCCUGUUUUCUUAGACGGA AGACCACUCCUGCAGAACAUUUAUUAUCAG CCUAGGCCAGUGUGAACAUACUGGGUACAU | 1 |
| | | UUUAGGAUUCAGUUUAUCUGUUUUGUUUA GGGUUGGUGCUAAUUUGGAAUAAGCAAAC CAUUCAUGAUCUGUAUCACAUAAUUUGGU UUAGAAUCUGUCACCUUACCUGUUGCGGUC ACUCCCUCUGAAUUUGGGGACCCCAAGACC AAGUUUUACUCUUCUGCUCUUUCCACCUCC AGUGCUAAGGCCUAGAGGGGCGCACCUUAA AUCUGAAUCAAUCCACAAAGCCCGACGGAC GCCAGUGGCAGGGUUUGGUGGGAUUUUAA UAGUAACCUACAAAAAAAGUCAGGAAGAU AGGGAAUAGGGACGAAUUAUUCUCUUCUA CUCUUCAAAUUUUCAGUGUUGCUCUCAUUG CAUAUUCUCACCUUGAAAUCACUGCUAGAG ACUUCCCAAGUCCAAAGGGUAAGAAACUAC CUUCAGACUGGCUUGGGAAAUGUUUAGAG CUGCCAGGGUUGUAUGUAAAAUUUCCAUG GCAAUAAACCACUGAUACAUUUUUUGUUCU AUUCCACCUAGGAAAAACAAAGAUCCCCAA AGUCCAUUUCGCUCCGAGGUCAGUGGACAC AGCUCUGUGACUGUCCUGUGCUGUGUGGGU CUGUAGAAGGGCCAGUCAUGGGCCAUCGAG CUACUCACAGCCAACUGCACCCAAGUCCAA GCCUGGCUCUGUUCCCAUACACAGAGACAG GAUGGGGGCUGCUGAUCUGGCCUGAAGCCU UGCUCUGGGCACUGAAAACGGGGGCUAUUU CCUUACCUGAAGAUCUGGAAGCAGCAUGUU GACAAAAGCACCAACUGCUCUGCCAUCACG UCCCACUGGGUGUCAGUGGGAAGGCCUAAC CAAGGCCUGCCUCCAGUGGCUAUCAGGCAG ACGGGCCAGCAGUUCCCCUCCAGGAGCAAG GGACCCUUCUUACCCUUUUGAUCACAUUUC CCUCUCUUAAAGGGGCACUGCUGUGGGAAG GGGUCAUGCCUUGGAUCCUACAGUCAUGGA | |
| | | CUGUGGGUGAAACAGGGGCUCUUGACUCUU CACUUCCUAGCCAUAACUCUGCCCAUUGGA CCACCCACCCUCCCUGCUGCUGGUCUCCUG GCUCACCCAUCAAAUCCAACUGCUUUAUCU AGAAAAUACUUUUCUCCAUUCUCAUUUCCC UUUGCUUUCCCACUCACCUCUGUUUUUGUU UCUGUUUUAUUUUGUUCCAGACACCCUUUU CAGGGAAGUAGAUCAGGGAGGCCCAAGAG AUUAGGCCUGGGUGGGCUGCUAUCGCUUAA GAACACGUCUGCCUCUGGGGCUCCUCCCUA ACAGCUGGCCUCUGGAGCUGGGAGAUGCAG CUGCCUGGCAGUCUGCAGAGGCAGAUUGUG UUGUUACGUGCUGGUCACCGCCUUGAGCCU UAAACCACAGUCAGCAAUGAACCUGGAGGC AGGGCAUCUCUGGGGAUGAUCGACCGCAUC UGCUCAGGUUCUUGCAGGGCAGGGCAGGCA GAGAUGUGUGUUUGGAGCCAGAAAGCCUU CCUGCCAACCUGCCAUGCCUCAGUUUCCCU AUCUGAACCACCUUCAUAAAAACAACUUAC GGCUCUCAGAGCAGCACAUUUGUAAAAACC UGAAAAAUAUAAAGAAAAACGUCACUCAC AUUUCUCCCAUGAACAUUUUCCUAUUUUAU GUAUCUUUUAUGUAACUGAAUCACAUUGC AUAUACAAUUAUGUGUUCCAUUUUUAAAC AAUAAGCUUCCCUUGUUUUAAAAGUAAUA AUCCAUGUUUAUUAUGUGUUAUUGCAUAG AAAUUUCAGACACAAAAAAAGAAAGGAAA AAUGACUCCUUAUUGCUCCAUUGAGAAAUA AUAAAUAUGAAUAUUAUUCUGAUAAAUA | |
| LNCRNA2 | AP000224.1 | AAUUUUUUUUUUUUUUUUUUUUUUGAGAG AAAGUCUCACUCUGUUGCCCAGGCUAGAGU GCAGUGGUACAGUCUUGGCUCACUGCAACC UCUGCCUCCCAGGUUCAAGCGAUUCUUGUG | 2 |
| | | UCUCAGCCUCCCAAGUAGUUGAGAUUACAG GCACGAGCCACCACACCUGGCGAAUUUUUG UAUUUUUAGUAGAGAGGGGGUUUCACCAU GUUGGCCAGGCUGGUCUCAACUCCUGACCU CAAGUAAUCCACCCAUCUCAGCCUCCCAAA GUGCUGGGAUUACAGGCGUGAGCCACAGCA CCUGCCCUGGUUGUCCUACUUUCAAUGAUG CUAAGAUUAAUCAGUGUUUCAGGUGAUAA CAGACUGAUCCUUCCAUUGUAAAGCUCCAA UCAACAUUUUGUCUAAUGAUUUUAGCCAU UAAUAAUCAUACCCUAAGUCAAUUACAUGC UGCAAAUGAUGAUUAGAUUCUAUUAUUUU UUCAUUAUCUAUUUUCUUUGACAUUCAUU AGCUUGAAUUCUAUAAAGAAGAACUUUUC CUUCUAAGUUAAGGCUAUGUGGUUACACA UACAUGCCUAAAAGAAAGCAGAAUAAAGA UAAGUAUUUAUUUUGAUUGCCAAAUAUCA GGGUAAGGAGUAACUGACCUAGUUAAUUC UAAUGGUGACCAAAGUGUGAUGACUUUUA AACAUUUUAUUGAUUUUCUUUUUUGUAUC UUUAUAAUUAAUGCAUUUUGAAAUAAAUU CAACACAUUUAAAUCAAUUGCAUUCAGUAU AUGUUUUCUCUUUUGACUCUCAUCUUGCCU CCAGUUUGGCUAAAGAAAGACUUUUUAGG UUAAUUCCUUUGUCCUUUGGGCACAAUCUC AUUAGUCUUUGAUAGCUACCUUGCUUUCUA AGUGAUAUGGUUAGGCUUUGUGUCCCUACC CAAAUCUCAUCUUGAAUUGUAAUCCCCAUA AUCCCCAUCAUCCCCACGUGUAAAGGGAGA GACGAGAUAGAGGUAACAAUCAUGGUGGC AGUUUCUCUCAUGCUGUUCUCAUGAUAGUG AGUUCUCACAAGAUCUGAUGGUUUUAUAA AGAGCUCUUCCCCCUUCACUUGGCAUUGUC | |
| | | CUUCUUGCCACCACUUUGUGAAGAAGGUGC CUUGCUUCCCCUUUGCCUUCUACCAUGAUU UUAAGUUUUCUGAGGCCUCCCCAGCCACAC UGAACUGUGAGUCAAUUAAACCUCUUUUCU UCAUAAAUUACCCAGUUUCUGGCAGUUCCU UAUAGCAGUGUGAAAACAGACUAAUACACC AGGGCAACAAACCGUCCCAGGCUCAUCUUA AAUUGCCUAACCUUAAAACUGGAGUCAGGU GUCCCUCCAAAGUUUCCUAGUCAUUGUUAG UGUGGAAUGGUAUUUAGGGAUUAUAGUCU AUAUGCUAAGUAUGCUCAUUUUCAAGGGG UUGUAGAAUAGUUUAUAUUAUUAUCCUCA ACUCCUAACUCCCUUCUCAUUUAUUAUUGU AUGAUUUCUAGGGCCUCCCAGCAGGCAGAA CACAAUUCUCCAUCCCAGUUCCACGUUUGG CCACGUGAUGUGCUUUGACCAAUGGGCACA UAUCAUGUUAAGUAGAAGCUUUUAGAGCU GUUAAGGACAGAGCUAAGCUUUGUCCUUU GCUUUUCCCUAGGCCACAAGGACAGUCUGU UCCAAACAGGGUCAAUUGCUUCAGCUUGAG UCCUGGAAAAAGAAGAUUGUAGAGAAAAG ACUCAGCAGCUGAUCUGGAGCCACCAAAAU GGAAUACAAGCCAAAUAUAUAAUGCAUAU GGUGGCAAACUACUGAGGUUUUUAAGGUU AUCUGUUACUUCUUCAAAGCUGAUUGAUAC AGGCUGUGAUGUCUUCUGGGCCUUUUUAG UAGACAGAUAGGAAAAGCAGCUUUGAAAA GAGGAAAGCAUUGUGAGUUCAAACUCAAU UCAAAUAGAACAUUUCCAAAUCAGAUAGA ACAUUAUAGGACCUGUACUUAAUUUUUAA AAACUUUAUACAUAUAUCUCUUUUUUCUAC ACUAAAAAUCUUAAUUUCUAUUUACAUUA GUAUAAUUACUGAUUUGUAUUUCCUAUAA | |
| | | UAUAACAUAGUGUCAAAAUAACAAAAUCA UUAUUUUAAAGUACUAAAGUUUAAAACAU AUUUGUAGCUAUUUUUGUCCUUAAGAUAU AUUCUACUGUUUGGUGGAAUAUUAUGUUU UUAGGUUCAGAUUGAAAUAAUUAUUUCCU UUAUGUGGUUAUAUCACUAAUUUGAUAUA CAGUAGGUUCAUUUCCUUAUUUCUUUUUU UUUCUUUUAGAGACAGCAUCUUUCUCUGUC ACCCACGCUAGAGUGCAGUAGCACAAUCAC AGCUCACCACAGCCUCGAUCUCCCAGGACC AAGCAAACCUCCCACCUCAGCCUCGUGAGU AGCUGAGACUACAGGCAUGCCACCAUGCCC AGCUAAUUUUUAUUUAUUUUUCUUUUUUG UAGAGGCGUGGCCUUCCUAUAUGGCCUAGG CUGGUCUUGAACUCCUGGUCUUAAGCAAUC UUCCUGCCUGGGCCUCCCAAAGUAUGGGGA UUACAAGUGUAAACCACCAUGCCCAGCUCA GUAAGUUCAUUUAUUUCCAUUUGUUUUCC AUUUUUAGGGAAGGCUAUUUUAGUGAAUU UUAUCUUUUGAAAUAAAACAUUUAAAAUU UCAAAAUGAAAUCUAUGUAAUAAGCUAUA CAUAGUAAGAGAAGUCUGUUCCAUUCCUGU UCUACCUAUUGUUAAUAGUUUCAUUAGUU AACAGUUUAACCUGCCAGUGUUUCUGUCAU ACACAAAAUGUAGCAUGUUGCACUAGAUU GUUCUGCAUCUUACUUUUUUCACUUACUAU AUUUUGGAGAUGACAGUACAGAGAGAUCA CCAUCAUUCCUUUAUAUCGCUACAGUGCCU ACCAUUGUGUUAUUUAUUUAGCCAAUCCCC UAUAAAUGGGCAUUUGUUUUCAAUCAUUU UUAUAAUAGUUGUGAAAUAAAUAAUACAA UGCAUAAAAAAGAAAUAACACCAUGCAUA AAGCAUGUCCUGUUUUUGCAACUACAGCUU | |
| | | UAACACAGGUUCCCAGUAACGGGGUUGAUG GGUCAAAGGGCAAAUAUAGAAUUUUUCUA CAUAUUGCUGAAUAUGAUGGGUCAAAGGC UAGAUGUAGAAUUUUUCUACAUAUUGCUG CUCCAUAGCAGUUGUAUCAUCUCACAGCUC CAUCAGCAGUAUGUAGAAUGCCUAUUUCCA UACAACUUCAAUAACAGAGUAUGUGGAUG AGUUUAUGUGAUUUUGCUAAGGGAAGAAG UGCUAUCUUUGGGACAAUCUAAUUUGCAU UUUAAGUUAUACUGAGAAAAGCUUCAUAU UUGAAGAACCAUUUAAAUUUUUCUUCUGG GAACUCUGGGUUUUUUGUGUGCCAUUUAU AAAAUUUUGAUAUUUGUAUUUUAAUUUUU AGGGGUUCCUUUUAUAUCAGUGAUAAUAC UCUGUCAGAGAUGUAACUUACAAAUAUCCU UUCCCAAUUAGUCAUUUAUCUUUGGAUUU GUUUAGGGUGUUUUAUUUUUUCCAUGCAG ACACUCAUUUGAUUUAUAGUCCAGUUUACA GAAGAACAGAUCCAUACUUUGUUUUUUU UUUUUUUUUUUUUUUGGCGGGGGGAGGUAU GGUUUUAUUUAUUUAUUUACCUAUUUACA UUUAGAUCUCUGAUCCAUUUGAAAUUAAU UCUCAUGGAGAAUAUCAGGUUCUGAUCCAU UUUUCCCUCAAAUGGUUCCCAGUUGUAACA AUAUUGAUUAAGCAUUCUCCAUGGUUUCA AAUGCUACCAUUUUACACACACAUACACAC AUACACGUGAUUUCUAAAUGAAGUUGGCA UAAUUUCCGGACUUUAUAUACUCUUCCAUU GGCUUGUCUGUCUAUUCAUGUACCAAUCUC ACAUUAUUUUACUUAUAGAGGCUUUAUAG AACACUAUAAUAUAGGGCUAUAACCACUUC AUUUCUGCCCCGUUAGUGUAUUAUUAAUUC UCCAUUGUCUCAUUCAUUUGAUAUUUAUUC | |
| | | UAUGUAUUAUAUGUUUUAUGAUUAAUAUG UUGAUACAGAAAUACAUAUUACAAUUUAU UAAAACACACGUCAGGACAUAUCCUCUUUU AAGAAAUGAAGUAGGAUAUGUAAUAUAAA AAAGUUUGAAGAUCACUGAUUCAGUGUUU UUUGAACAUGCCAGCAUCUUUAGAUAGCUG UCCUGGGUGGCAGUAGCACAAUGGAGCUGA GAUUCUUACAUGUAUCUGAUUUUGGCAUU UGCCUAAUGCUGAAUGAGAUUCUUGGCAG AGACAAUGACUCACCUGGAUCCAGGGGUAA UUUUUUAAGUGCUAAUGAGCAAGUGUCAA GAUCCCACAAAAUCAUUAAAAGUAACGGCU AAAACUGCAGUUACUUUUGCACCAACCUAU AAAAAUACUGGACAAGAACACCUAGGAAA AUCAUCUUUGAUUUGGAAAUGAAGAUCAC UUUAUCAGGCUCUACAAUGGUUUAAAUGU GUUCCUCAGAAUUCAUGUAUAGGAAAUUG AGUUCCCAGUAUAAUGGUGUUGAGAGGUG GUAGGACCUUUAAGAGGUGUUUGGGUCAU GAAGACUCUGCCACCCUCACGAAGAUAUUA GUCCCAUUCUCAUAGGACUGGGUUAAUUCU CACAGAAGCAAAUGAGUUCUUGUUCUCAUG GGACUAGAUUAGUAACUGCAAGAGCAGGU UGUUAAAAAAUGAGGCCAUCACUUGUGUU UUGUCUCUUUUGCACAUACUUGCUUGCUGC UCCUGCAGGUGUUUCCACAUGUGAUGCCAU UCACUAUGCUUUAAUGUAGCAUGAGGGCCU UGCCAGAGGUAGCUGCCCAAUGUUGAACUU CCCAGGCUCCAGAACUGGGAGCCAAGUAAA CCUCUUUUGUUUAUAAAUUACCCAGUCUCA GGUGUUCUGUUACAGCAACAGAAAUAGAC UAAAACAGUCUCUAUUGUCUCAUCUCUCUU AAUAAACUUUUGCUCAUUAUGGGAUAUAG | |
| | | AUUGCUAUUAUAUCAAUUGAGUAACAAAG AGAUUAGGCCAUGAGGGGAAAGUCUGGAG AUUCCCUGCUCCAGAGAGUAGAACUUUUAC AACUAACAUGCUAAUACGGUUUGGUUGUG UCGCCACCCAAAUCUCAUCUUGAAUUGUAG UUCCCAUAAUCCCCACAUGUUGUGGGAGAG ACACGGUGGCAGGUAAUUGAAUCAUGGAA GUGGUUACCUCCAUGCUUUCUUUUCUCAUG AUAGUGAAUGAGCUCUUAUGAGAUCUGAU GAUUUUAUAAAGGGCUUUCCCCGCUUUUGC UCGGCACUUCUGCUUCCGGCCAUCAAGUAA AGAAGAAUGUGUUUGCUUCCCCUUCUGCCA UGAUUGUAAGUUUCCUGAGGCCUCCCCAGC CAUGCGAAACUGAGAGUCAAUUAAACCUCU UUCCUUUGUAAAUUACCCAGUCUUGGGCAG UCCUUUACAGCAGCGUGAGAACACACUAAU ACGUGCUUUGUUCUAGUCUAGAUUAAGCA ACUUUUGAGGGGUGUAUUGUUUGACUCAU CUGAGUUUUUAAUCAAUGCUUACUAAUUC AAGAGGAACUUAUGUGCUUUUAGGAUAUA AGGAGGCAUCAGGAAAUCUGGAAAAUUUC UUAAAAUCCUAUAAGGCAGACCAGUCUUUA UAUUUCCUUAUAUCUUAGGGAGGCCACUGU UAAAAAGAGGAGGAGGGAGUUUUAGGAGC AGUAAUAUUAUGCAUUCAUUUGAGACUAU UAAUUUUACAUAUGUCUUGAGGUUUUUAU UGCUAAAAAGUUAUAUAAUUUUAUGAAGC UACAAAACAAAUAAACAUUAAAUUCCAGCU UAUGUAAUUCUACUUAAUCAAGUAUUAAA UGUGCCAGGCAGUACUCUAGGUACCAACGA CCUAAAGAUGUGUUGUCUCUGCCUCCAGCA AGCUCACAAUACAGAGAGAUAUACAUGGA AAAAUUGUAACAGUACAGCUCAGUAAGUG | |
| | | CCAUAAGAGAAUAAAGAAUAUACCAAAAG CACCAGAGUAUAAUAUACAGUAGACAUCAG CUUUGAGGAAUUGAUGAGUUGGAAAGAAU UAAGAGAAGGCGCAAGGAAUUUUGGAGUU AGACCUUCAGGGAGAAAUUGCAUUUGGCCA UAUGAGGAACAAUAGCAACAACAACAGAG AUGAAUGGAGGUUGGGAAGAAGUACUGUA UCUAGAUAAAAUGGCAUAGAUGUUCACUC GUUUACACACACACACACACACACACACAC AAACACACGGCACAAAAGAACCUGACUUGU UCUGGAUCCAAAAGCACUAUAUGGACAGUA CAAUAAUCUCAUGUCUCCAAUAUGCUUAGG UACAAUUUUAAUGUUUUAUGACAUAGAUG GCUGCAUGUUUCAGUUAUUGGACAAAUGC AUACUUGAGUUAUUGUUUAUUCCAUUGUG UCAGUUCUGCUUCUGAUCAAAAUCCAUUCC UAUUUGAAAAUGCAUCUAGUGAAUUUUUUU UUUUGAGACAGAAUCUUGCUGUGUUGCCC AGGCUGGAGUGCAGUGGCACAAUCUCAGCU CAUUGCAACCUCCGCCUCCCACGUUCAAGU GAUUCUCAUGCCUCAGCCUCCUGAGUAGCU GGGACUACAGGUGCAUGCCACCAUGCCCAG CUACUUUUUGUACUUUUAGUAGAUACAGG GUUUCACCACGUUGGCUAGGCUGGUUUCAA ACUCCUGACCUCAGGUGAUCCACCCGUCUU GGCCUCCCAAAGUGCUGGGAUUACAGGCGU GAGUCACAGCGCCCAGCCCUCUAGUGCAUU UUUUAAAAAACAUAAACACCAUUCUCUCUU UUCAAAUUUAGCACUUGCCCAGAAGUUUGA CAUGUUCCCUUUUUAUUUGUGGUCAUUAU UUCCUCUUUGGUCUCCUUUAAAAAUAGCAC CCCUUUGCACUUGUCUGUGAAUCUUAGCUG GUGCUAGUGUCUCUGUCCUCUCUGGGUGCA | |
| | | CACACGUUCUCCUUUGGACUUUUUAACUGG AUCUUGCUUGGAAUUUUUCCCUUUCCAUAU UUGGUUACUGGUUUGGAAGUUUAUUUCCC UUUGUUUUACUAAUCAUUUUGUUCCAUUA UUUUUGGUGGUUCUAGUUAGAAAAUAGCU CCCACUGCAUUUUGGUGCAACAAUUUUAAA GGCCUCUAUAAGAUGUUAUCUUCUGUCCUG CUUGUAGACUGAGCUUUAGGUUGUACCUU AAUUACUAACAUAAAUAACUUUUUGGAGC AAGGUGCACAACACUAUAUAUAAUAUGAU UUGAUUUUCUGGAAGGUAUACAGCAGUAA GCAUAAUAAAUUGUGAUCUUUAUUUCUAA AGAAAUUAUAAGAGUAUGCUUAUAUAUAC UGGAACUAUUUCUUUUCUAAUUUCCUGAA ACCAUUUGCAGGAAACUUAAGGAUGGUCU AUCUGAGAAGCAAAGGAGUCAGGAAGGCU GGAAAGGAGACUUAUUUCUUGUUUUGUAA ACUUACUGCUCUGUUUGAAUUUACCCUAAG CAUAUAUUAUUUUUAGUUAGACAUAUAAG AAAAUGUGUUUAACAAUUCAUUCAUUUUU AUCUUUGGUUUUGUCCAUAGUAUGUAUUU AGGAAGUUUCACAAUGUGAAGAAAGCGAA GAGUAAAAACAGCAGGUGACUGCAUUGAC AAAAUGAAAGCAAGCAAAUUGGACAAACC UAGCAUAAAGAAGACAGACCAGGUUAUGC AUUUUUACUCCUUACAAAUAUAAAGACAA GCCCGUCCUAAAUUAUCCCAGUAGAAAAUU UUGACAACUACAACAAAACAGAAAACCCAA CAAAACAAAUAAAAAUUUGAUGCUGAUGA CAUGCGAUAAAGAAAAGCAGAGCUCUCCAG UAAAAACCAAAAUUCCUGUCUGUUAAGUU UACAAGGAUUCUUCCUUAGGACUUUCUGAG CCAAGACAAUGGACAGCUAAGAGGGCAGCU | |
| | | CUUGAAAUACUUUCACAGGAUUUGCUUCUC UGCUCAUAAAAUGUAGGAACAAUUCAAAC UGGAGAGCAUUUCCUACCCAUUCACUGGCC UGAAGAUUCAUAGAGUAGGGUUUUGUGUU GCUGUCAGCAGGCAUUUAUCUGAGCACACA UCCAUCCAUCCUCCCUCUCUCUCUCUCUCU CCUUCUCUCUCUCUCUCUUUCCCCCCACAC CCGAUGCAUGCAUACACUCACACACUUUGG AACUUAGAAGGCCACUCUUCUGAAUAUCAA AUCAAGUAAAAUGACCAUUCUAAGGGUGA CAACUCAGGGAAACUCUGGAAACACUGGAA ACAAAUACUGAAAAUAAACUUACGUGUGG GAGAGAGAAUAACAUUGAAAGAAGGUAUA CAUAGUAACCAAAAGAGCUACUGUUUCAUG UUUAUAGGUUGGGAACUGAACAGAAAGUU GAAAACACAGCACACAUGAUUAAAAAAUU UUUUCCUUAAAAACCAAAGAGCCAUUUUUC UUAAAUUCCUGAUCAGAUUUAAUUAACUA CAUUUCAAAUGCAGUCAUGCACUGCAUAAU GACAUUUUGGUCAACAAUGAACCAUAUCAG UGGUCCCACAGAUUAUAAUGGAGCUGAAA AAUUCUUAUCAUCUAGUGAUGCUAUAACGC CCGUACU | |
| LNCRNA3 | AC098484.1 | UAGUCAGAAUCCACUACUCUACCACUAAUG UCUCUUCCAUCCGGCUCUGCAGCACCUGAG CAAAAGACCUCUGCAGCUGAGUCCAAAGAG CAUUCCCAUGUGAACCGAGCAACCAAGUAU CAUCGGAGUGAGUUUGUUAGAUUCUAGCCC CAUCCUGUGCUAGGCCUUAGGUAGACCUCU UACAGCUCACAAGAAAUGCAGUUUAUGAG GAAAGACCAUGAAUCGGGGGGCGGCCCGGG GAGGGUAUUACCAAACAGAUCAGCAUCAGG AACAUCAUUCCCAGAGAGAAAUUUGGCCAA | 3 |
| | | UUGUCAGUUCAAGUACACAUCUCUCCCUCC UUUUACAGCAUAUUGCAAUAGAUUAUAGC CAUGCGAAAUAAUCCAUAAACUUAGCUAAU UCCACCAAACAGAUAGGAAAGAAGGUAGA GUCCUACAAGAGCCUAGUCAGAAUAAGAAC UACAGUAAUUUUGCCAAAACCCUGUUAAGU UCUUGAAUAAUUCUGGUCAAAAUGGAUUU CAAUCUGAGUUAACUCACAGUCAACAAUUU GGCUGUUUUUCUUGCUUCUCACUCUACUGC CUUACUCUCUUCCACAGUGUUAAGUAGCCA UUUAAAUGGGUUUGUUCUGCUGCAGGUGG CUGAGCCUGGGAUUUCCAACUUGGGAGACU ACACAGUGGACUCUAUAAUGAUUAGUGGC AGCAUCCAAUGUCUCUUCCAAGUCUAAAAU UUGUGAGUUUCAAAGUUGACUCUCCCCAAA CCUGGCCUUUGCUGCCUCAGAUUCUGUCAC UCUAGCAAGGCAGAGAGAGCAAAAGGGGC AAGACAAACCUCAUCAACAGCACAGAAAUC CCAGAAUCACACACUCUCUGGUCCAGCCAA GCCAAGUGUUUUUUUUUUUGUUGUUGUUG UUGUUUUUUAAAUCACCCUCCAUUACUCAA CAUUCUCGUUCCCAGAUGAAUUUUAAACUG GAGUUGCACCAACUGUGAGACAGCCU | |
| LNCRNA4 | AC124283.1 | CUGGGUUCAAGAUGCAGAAUGGUGCCUACG GCAAAAAAAAAAAAAAAAAACACCCUCCUC CCUUCUUUACCAUUUGAAUGGACAUUUUCC UUACCUGUGAUCCCAACAGAAACAGAUCCA GACCUAUCAUGUGAAGUCCACGUUCCAGGA UCAGAAGUAACCAGUUUAUGGACUGAGCU UACACGGGAAAGUCUACCCCCGACUCCUUC UGGAUAGUAACAUACACAGCUGCAUAAAA ACGUCUCCAAGGGGACAUACGAUGCAUUUG CUUGGUGUCCCAGCCAAGCUCCCCACCGGC | 4 |
| | | GACCUCACUGUUCCUUAGAGCUCAAGAGCU CGUCUCCUAUCAAUCAGAGAACCCCAUCAG CUGUGACCAACAGAGCUGGAGCCCUCUGUG GAGGGAGCUGACCCCACACACAGGACAGAG CAGAAUCCUGAUUAUUUUACAAACUGCAAA CCUUCUGAGUAAGAAGACAAAAAUAUACA UUCCAAGGUAUCUGUAAAGUGCUUGGAAG AUGCAGACAGCUGCACCGAGGGGCUCUGAU CCAUCCACACGCUGCGCUUUGCUGCGGUCA CACACACGGUCUCAGUCACGUGAUGGUUUU GCUUUUAUUUCUUAAACGGCUGAGUGAUA AUCCAGCUAGUGUGCAGUCAUUUCAUACCU UUCAAUGGGCGUCACCGCAGUGACGCUGCC CCAGCCCCAUGCUGAGGGCCGACACAAUUC ACGGAACAGAUUCAUCAUAUUUGGUCUUU AUGUAAAUAAUAAAUGUUUUAAAAUUGCC UAAAUAUA | |

### Example 3: Description of cDNA sequences obtained by reverse transcription from the pCHIKV-CIJD biomarkers

| Lnc-RNA# | Reference | NUCLEIC ACID SEQUENCE | SEQ ID NO: |
|---|---|---|---|
| LNCRNA1 | AC104561.4 | AGGACCGCCGAGCTTTGCTGAGGCATCATGGTGG GACTTAAAGAAGTATCTTGGCCGGGCGCAGTGGC TCACACCTATAATCCCAGAACTTTGGGAGGATGA GGCAGGAGGATCAATTGAGTCACGGAGTTCAAG ACCAGCCTGGGCAACAGAGTGAGACTCTGTCTCT ACAACAAATTTTAAAAAAATTAGTCAGGTATGGT GACGTGCACGTATAGTCTCAGCTACTTGGGAGGC | 5 |
| | | TGAGGTGGGAGGATCACTTGAGCTCGGGAGGTG GAGGTTGCAGTGAGCCATGATTGCGCCACTCACT CTAACATAGGTGACAGAGTGAGATCCCGTCTCCA AAAACAAACACAAACAAACTAAAAGAAGTATCA GCTGCTACACATCTGGAATGACAACATATGCAGA ATACAAATGTAAATAGAAAACATAGAATATAAA TTATGAGTTTTAGTTGAGTAACGCTGACTTGGAA TTGGAAGAACATTTGCAGGCATCAGTAATGGGAG AGTAGAAAATGTGAGAAAGAAACCTTAAGTCAA TTAAGAGAATACACTCTGCAAATTAGGGGTGGAT CCTTGGGTTAATCTGCAGCCTGCAGTTGTACCCTG CTAGAAGCAGCTTCGATGCCACTGAAGTTAATCT TGGGTCCCAGTAAATATCTTCTCTCCTCTCTATTA TCCACTGTGCAAGATATATGTAAATACTGCCAGA TACATTTTTTAAAAGGCCCGGGTGCTATCGTGTAT AGGTTGACATTAATGTACTCCCAGAATGCCCAGG CATGGTATGGGGTGGCCACCCTATGGTTCCTGTTT TCTTAGACGGAAGACCACTCCTGCAGAACATTTA TTATCAGCCTAGGCCAGTGTGAACATACTGGGTA CATTTTAGGATTCAGTTTATCTGTTTTGTTTAGGG TTGGTGCTAATTTGGAATAAGCAAACCATTCATG ATCTGTATCACATAATTTGGTTTAGAATCTGTCAC CTTACCTGTTGCGGTCACTCCCTCTGAATTTGGGG ACCCCAAGACCAAGTTTTACTCTTCTGCTCTTTCC ACCTCCAGTGCTAAGGCCTAGAGGGGCGCACCTT AAATCTGAATCAATCCACAAAGCCCGACGGACGC CAGTGGCAGGGTTTGGTGGGATTTTAATAGTAAC CTACAAAAAAAGTCAGGAAGATAGGGAATAGGG ACGAATTATTCTCTTCTACTCTTCAAATTTTCAGT GTTGCTCTCATTGCATATTCTCACCTTGAAATCAC TGCTAGAGACTTCCCAAGTCCAAAGGGTAAGAAA CTACCTTCAGACTGGCTTGGGAAATGTTTAGAGC TGCCAGGGTTGTATGTAAAATTTCCATGGCAATA AACCACTGATACATTTTTTGTTCTATTCCACCTAG GAAAAACAAAGATCCCCAAAGTCCATTTCGCTCC GAGGTCAGTGGACACAGCTCTGTGACTGTCCTGT | |
| | | GCTGTGTGGGTCTGTAGAAGGGCCAGTCATGGGC CATCGAGCTACTCACAGCCAACTGCACCCAAGTC CAAGCCTGGCTCTGTTCCCATACACAGAGACAGG ATGGGGGCTGCTGATCTGGCCTGAAGCCTTGCTC TGGGCACTGAAAACGGGGGCTATTTCCTTACCTG AAGATCTGGAAGCAGCATGTTGACAAAAGCACC AACTGCTCTGCCATCACGTCCCACTGGGTGTCAG TGGGAAGGCCTAACCAAGGCCTGCCTCCAGTGGC TATCAGGCAGACGGGCCAGCAGTTCCCCTCCAGG AGCAAGGGACCCTTCTTACCCTTTTGATCACATTT CCCTCTCTTAAAGGGGCACTGCTGTGGGAAGGGG TCATGCCTTGGATCCTACAGTCATGGACTGTGGG TGAAACAGGGGCTCTTGACTCTTCACTTCCTAGC CATAACTCTGCCCATTGGACCACCCACCCTCCCT GCTGCTGGTCTCCTGGCTCACCCATCAAATCCAA CTGCTTTATCTAGAAAATACTTTTCTCCATTCTCA TTTCCCTTTGCTTTCCCACTCACCTCTGTTTTTGTT TCTGTTTTATTTTGTTCCAGACACCCTTTTCAGGG AAGTAGATCAGGGAGGCCCAAGAGATTAGGCCT GGGTGGGCTGCTATCGCTTAAGAACACGTCTGCC TCTGGGGCTCCTCCCTAACAGCTGGCCTCTGGAG CTGGGAGATGCAGCTGCCTGGCAGTCTGCAGAGG CAGATTGTGTTGTTACGTGCTGGTCACCGCCTTGA GCCTTAAACCACAGTCAGCAATGAACCTGGAGGC AGGGCATCTCTGGGGATGATCGACCGCATCTGCT CAGGTTCTTGCAGGGCAGGGCAGGCAGAGATGT GTGTTTGGAGCCAGAAAGCCTTCCTGCCAACCTG CCATGCCTCAGTTTCCCTATCTGAACCACCTTCAT AAAAACAACTTACGGCTCTCAGAGCAGCACATTT GTAAAAACCTGAAAAATATAAAGAAAAACGTCA CTCACATTTCTCCCATGAACATTTTCCTATTTTAT GTATCTTTTATGTAACTGAATCACATTGCATATAC AATTATGTGTTCCATTTTTAAACAATAAGCTTCCC TTGTTTTAAAAGTAATAATCCATGTTTATTATGTG TTATTGCATAGAAATTTCAGACACAAAAAAAGAA AGGAAAAATGACTCCTTATTGCTCCATTGAGAAA | |
| | | TAATAAATATGAATATTATTCTGATAAATA | |
| LNCRNA2 | AP000224.1 | AATTTTTTTTTTTTTTTTTTTTTTGAGAGAAAGTCT CACTCTGTTGCCCAGGCTAGAGTGCAGTGGTACA GTCTTGGCTCACTGCAACCTCTGCCTCCCAGGTTC AAGCGATTCTTGTGTCTCAGCCTCCCAAGTAGTT GAGATTACAGGCACGAGCCACCACACCTGGCGA ATTTTTGTATTTTTAGTAGAGAGGGGGTTTCACCA TGTTGGCCAGGCTGGTCTCAACTCCTGACCTCAA GTAATCCACCCATCTCAGCCTCCCAAAGTGCTGG GATTACAGGCGTGAGCCACAGCACCTGCCCTGGT TGTCCTACTTTCAATGATGCTAAGATTAATCAGTG TTTCAGGTGATAACAGACTGATCCTTCCATTGTA AAGCTCCAATCAACATTTTGTCTAATGATTTTAGC CATTAATAATCATACCCTAAGTCAATTACATGCT GCAAATGATGATTAGATTCTATTATTTTTTCATTA TCTATTTTCTTTGACATTCATTAGCTTGAATTCTA TAAAGAAGAACTTTTCCTTCTAAGTTAAGGCTAT GTGGTTACACATACATGCCTAAAAGAAAGCAGA ATAAAGATAAGTATTTATTTTGATTGCCAAATAT CAGGGTAAGGAGTAACTGACCTAGTTAATTCTAA TGGTGACCAAAGTGTGATGACTTTTAAACATTTT ATTGATTTTCTTTTTTGTATCTTTATAATTAATGCA TTTTGAAATAAATTCAACACATTTAAATCAATTG CATTCAGTATATGTTTTCTCTTTTGACTCTCATCTT GCCTCCAGTTTGGCTAAAGAAAGACTTTTTAGGT TAATTCCTTTGTCCTTTGGGCACAATCTCATTAGT CTTTGATAGCTACCTTGCTTTCTAAGTGATATGGT TAGGCTTTGTGTCCCTACCCAAATCTCATCTTGAA TTGTAATCCCCATAATCCCCATCATCCCCACGTGT AAAGGGAGAGACGAGATAGAGGTAACAATCATG GTGGCAGTTTCTCTCATGCTGTTCTCATGATAGTG AGTTCTCACAAGATCTGATGGTTTTATAAAGAGC TCTTCCCCCTTCACTTGGCATTGTCCTTCTTGCCA CCACTTTGTGAAGAAGGTGCCTTGCTTCCCCTTTG | 6 |
| | | CCTTCTACCATGATTTTAAGTTTTCTGAGGCCTCC CCAGCCACACTGAACTGTGAGTCAATTAAACCTC TTTTCTTCATAAATTACCCAGTTTCTGGCAGTTCC TTATAGCAGTGTGAAAACAGACTAATACACCAGG GCAACAAACCGTCCCAGGCTCATCTTAAATTGCC TAACCTTAAAACTGGAGTCAGGTGTCCCTCCAAA GTTTCCTAGTCATTGTTAGTGTGGAATGGTATTTA GGGATTATAGTCTATATGCTAAGTATGCTCATTTT CAAGGGGTTGTAGAATAGTTTATATTATTATCCTC AACTCCTAACTCCCTTCTCATTTATTATTGTATGA TTTCTAGGGCCTCCCAGCAGGCAGAACACAATTC TCCATCCCAGTTCCACGTTTGGCCACGTGATGTGC TTTGACCAATGGGCACATATCATGTTAAGTAGAA GCTTTTAGAGCTGTTAAGGACAGAGCTAAGCTTT GTCCTTTGCTTTTCCCTAGGCCACAAGGACAGTCT GTTCCAAACAGGGTCAATTGCTTCAGCTTGAGTC CTGGAAAAAGAAGATTGTAGAGAAAAGACTCAG CAGCTGATCTGGAGCCACCAAAATGGAATACAA GCCAAATATATAATGCATATGGTGGCAAACTACT GAGGTTTTTAAGGTTATCTGTTACTTCTTCAAAGC TGATTGATACAGGCTGTGATGTCTTCTGGGCCTTT TTAGTAGACAGATAGGAAAAGCAGCTTTGAAAA GAGGAAAGCATTGTGAGTTCAAACTCAATTCAAA TAGAACATTTCCAAATCAGATAGAACATTATAGG ACCTGTACTTAATTTTTAAAAACTTTATACATATA TCTCTTTTTTCTACACTAAAAATCTTAATTTCTATT TACATTAGTATAATTACTGATTTGTATTTCCTATA ATATAACATAGTGTCAAAATAACAAAATCATTAT TTTAAAGTACTAAAGTTTAAAACATATTTGTAGC TATTTTTGTCCTTAAGATATATTCTACTGTTTGGT GGAATATTATGTTTTTAGGTTCAGATTGAAATAA TTATTTCCTTTATGTGGTTATATCACTAATTTGAT ATACAGTAGGTTCATTTCCTTATTTCTTTTTTTTTC TTTTAGAGACAGCATCTTTCTCTGTCACCCACGCT AGAGTGCAGTAGCACAATCACAGCTCACCACAGC CTCGATCTCCCAGGACCAAGCAAACCTCCCACCT | |
| | | CAGCCTCGTGAGTAGCTGAGACTACAGGCATGCC ACCATGCCCAGCTAATTTTTATTTATTTTTCTTTTT TGTAGAGGCGTGGCCTTCCTATATGGCCTAGGCT GGTCTTGAACTCCTGGTCTTAAGCAATCTTCCTGC CTGGGCCTCCCAAAGTATGGGGATTACAAGTGTA AACCACCATGCCCAGCTCAGTAAGTTCATTTATTT CCATTTGTTTTCCATTTTTAGGGAAGGCTATTTTA GTGAATTTTATCTTTTGAAATAAAACATTTAAAAT TTCAAAATGAAATCTATGTAATAAGCTATACATA GTAAGAGAAGTCTGTTCCATTCCTGTTCTACCTAT TGTTAATAGTTTCATTAGTTAACAGTTTAACCTGC CAGTGTTTCTGTCATACACAAAATGTAGCATGTT GCACTAGATTGTTCTGCATCTTACTTTTTTCACTT ACTATATTTTGGAGATGACAGTACAGAGAGATCA CCATCATTCCTTTATATCGCTACAGTGCCTACCAT TGTGTTATTTATTTAGCCAATCCCCTATAAATGGG CATTTGTTTTCAATCATTTTTATAATAGTTGTGAA ATAAATAATACAATGCATAAAAAAGAAATAACA CCATGCATAAAGCATGTCCTGTTTTTGCAACTAC AGCTTTAACACAGGTTCCCAGTAACGGGGTTGAT GGGTCAAAGGGCAAATATAGAATTTTTCTACATA TTGCTGAATATGATGGGTCAAAGGCTAGATGTAG AATTTTTCTACATATTGCTGCTCCATAGCAGTTGT ATCATCTCACAGCTCCATCAGCAGTATGTAGAAT GCCTATTTCCATACAACTTCAATAACAGAGTATG TGGATGAGTTTATGTGATTTTGCTAAGGGAAGAA GTGCTATCTTTGGGACAATCTAATTTGCATTTTAA GTTATACTGAGAAAAGCTTCATATTTGAAGAACC ATTTAAATTTTTCTTCTGGGAACTCTGGGTTTTTT GTGTGCCATTTATAAAATTTTGATATTTGTATTTT AATTTTTAGGGGTTCCTTTTATATCAGTGATAATA CTCTGTCAGAGATGTAACTTACAAATATCCTTTCC CAATTAGTCATTTATCTTTGGATTTGTTTAGGGTG TTTTATTTTTTCCATGCAGACACTCATTTGATTTA TAGTCCAGTTTACAGAAGAACAGATCCATACTTT GTTTTTTTTTTTTTTTTTTTTTTGGCGGGGGGAGGT | |
| | | ATGGTTTTATTTATTTATTTACCTATTTACATTTAG ATCTCTGATCCATTTGAAATTAATTCTCATGGAGA ATATCAGGTTCTGATCCATTTTTCCCTCAAATGGT TCCCAGTTGTAACAATATTGATTAAGCATTCTCCA TGGTTTCAAATGCTACCATTTTACACACACATAC ACACATACACGTGATTTCTAAATGAAGTTGGCAT AATTTCCGGACTTTATATACTCTTCCATTGGCTTG TCTGTCTATTCATGTACCAATCTCACATTATTTTA CTTATAGAGGCTTTATAGAACACTATAATATAGG GCTATAACCACTTCATTTCTGCCCCGTTAGTGTAT TATTAATTCTCCATTGTCTCATTCATTTGATATTT ATTCTATGTATTATATGTTTTATGATTAATATGTT GATACAGAAATACATATTACAATTTATTAAAACA CACGTCAGGACATATCCTCTTTTAAGAAATGAAG TAGGATATGTAATATAAAAAAGTTTGAAGATCAC TGATTCAGTGTTTTTTGAACATGCCAGCATCTTTA GATAGCTGTCCTGGGTGGCAGTAGCACAATGGAG CTGAGATTCTTACATGTATCTGATTTTGGCATTTG CCTAATGCTGAATGAGATTCTTGGCAGAGACAAT GACTCACCTGGATCCAGGGGTAATTTTTTAAGTG CTAATGAGCAAGTGTCAAGATCCCACAAAATCAT TAAAAGTAACGGCTAAAACTGCAGTTACTTTTGC ACCAACCTATAAAAATACTGGACAAGAACACCTA GGAAAATCATCTTTGATTTGGAAATGAAGATCAC TTTATCAGGCTCTACAATGGTTTAAATGTGTTCCT CAGAATTCATGTATAGGAAATTGAGTTCCCAGTA TAATGGTGTTGAGAGGTGGTAGGACCTTTAAGAG GTGTTTGGGTCATGAAGACTCTGCCACCCTCACG AAGATATTAGTCCCATTCTCATAGGACTGGGTTA ATTCTCACAGAAGCAAATGAGTTCTTGTTCTCAT GGGACTAGATTAGTAACTGCAAGAGCAGGTTGTT AAAAAATGAGGCCATCACTTGTGTTTTGTCTCTTT TGCACATACTTGCTTGCTGCTCCTGCAGGTGTTTC CACATGTGATGCCATTCACTATGCTTTAATGTAGC ATGAGGGCCTTGCCAGAGGTAGCTGCCCAATGTT GAACTTCCCAGGCTCCAGAACTGGGAGCCAAGTA | |
| | | AACCTCTTTTGTTTATAAATTACCCAGTCTCAGGT GTTCTGTTACAGCAACAGAAATAGACTAAAACAG TCTCTATTGTCTCATCTCTCTTAATAAACTTTTGCT CATTATGGGATATAGATTGCTATTATATCAATTG AGTAACAAAGAGATTAGGCCATGAGGGGAAAGT CTGGAGATTCCCTGCTCCAGAGAGTAGAACTTTT ACAACTAACATGCTAATACGGTTTGGTTGTGTCG CCACCCAAATCTCATCTTGAATTGTAGTTCCCATA ATCCCCACATGTTGTGGGAGAGACACGGTGGCAG GTAATTGAATCATGGAAGTGGTTACCTCCATGCT TTCTTTTCTCATGATAGTGAATGAGCTCTTATGAG ATCTGATGATTTTATAAAGGGCTTTCCCCGCTTTT GCTCGGCACTTCTGCTTCCGGCCATCAAGTAAAG AAGAATGTGTTTGCTTCCCCTTCTGCCATGATTGT AAGTTTCCTGAGGCCTCCCCAGCCATGCGAAACT GAGAGTCAATTAAACCTCTTTCCTTTGTAAATTAC CCAGTCTTGGGCAGTCCTTTACAGCAGCGTGAGA ACACACTAATACGTGCTTTGTTCTAGTCTAGATTA AGCAACTTTTGAGGGGTGTATTGTTTGACTCATCT GAGTTTTTAATCAATGCTTACTAATTCAAGAGGA ACTTATGTGCTTTTAGGATATAAGGAGGCATCAG GAAATCTGGAAAATTTCTTAAAATCCTATAAGGC AGACCAGTCTTTATATTTCCTTATATCTTAGGGAG GCCACTGTTAAAAAGAGGAGGAGGGAGTTTTAG GAGCAGTAATATTATGCATTCATTTGAGACTATT AATTTTACATATGTCTTGAGGTTTTTATTGCTAAA AAGTTATATAATTTTATGAAGCTACAAAACAAAT AAACATTAAATTCCAGCTTATGTAATTCTACTTAA TCAAGTATTAAATGTGCCAGGCAGTACTCTAGGT ACCAACGACCTAAAGATGTGTTGTCTCTGCCTCC AGCAAGCTCACAATACAGAGAGATATACATGGA AAAATTGTAACAGTACAGCTCAGTAAGTGCCATA AGAGAATAAAGAATATACCAAAAGCACCAGAGT ATAATATACAGTAGACATCAGCTTTGAGGAATTG ATGAGTTGGAAAGAATTAAGAGAAGGCGCAAGG AATTTTGGAGTTAGACCTTCAGGGAGAAATTGCA | |
| | | TTTGGCCATATGAGGAACAATAGCAACAACAACA GAGATGAATGGAGGTTGGGAAGAAGTACTGTAT CTAGATAAAATGGCATAGATGTTCACTCGTTTAC ACACACACACACACACACACACACAAACACACG GCACAAAAGAACCTGACTTGTTCTGGATCCAAAA GCACTATATGGACAGTACAATAATCTCATGTCTC CAATATGCTTAGGTACAATTTTAATGTTTTATGAC ATAGATGGCTGCATGTTTCAGTTATTGGACAAAT GCATACTTGAGTTATTGTTTATTCCATTGTGTCAG TTCTGCTTCTGATCAAAATCCATTCCTATTTGAAA ATGCATCTAGTGAATTTTTTTTTTTGAGACAGAAT CTTGCTGTGTTGCCCAGGCTGGAGTGCAGTGGCA CAATCTCAGCTCATTGCAACCTCCGCCTCCCACGT TCAAGTGATTCTCATGCCTCAGCCTCCTGAGTAG CTGGGACTACAGGTGCATGCCACCATGCCCAGCT ACTTTTTGTACTTTTAGTAGATACAGGGTTTCACC ACGTTGGCTAGGCTGGTTTCAAACTCCTGACCTC AGGTGATCCACCCGTCTTGGCCTCCCAAAGTGCT GGGATTACAGGCGTGAGTCACAGCGCCCAGCCCT CTAGTGCATTTTTTAAAAAACATAAACACCATTC TCTCTTTTCAAATTTAGCACTTGCCCAGAAGTTTG ACATGTTCCCTTTTTATTTGTGGTCATTATTTCCTC TTTGGTCTCCTTTAAAAATAGCACCCCTTTGCACT TGTCTGTGAATCTTAGCTGGTGCTAGTGTCTCTGT CCTCTCTGGGTGCACACACGTTCTCCTTTGGACTT TTTAACTGGATCTTGCTTGGAATTTTTCCCTTTCC ATATTTGGTTACTGGTTTGGAAGTTTATTTCCCTT TGTTTTACTAATCATTTTGTTCCATTATTTTTGGTG GTTCTAGTTAGAAAATAGCTCCCACTGCATTTTG GTGCAACAATTTTAAAGGCCTCTATAAGATGTTA TCTTCTGTCCTGCTTGTAGACTGAGCTTTAGGTTG TACCTTAATTACTAACATAAATAACTTTTTGGAGC AAGGTGCACAACACTATATATAATATGATTTGAT TTTCTGGAAGGTATACAGCAGTAAGCATAATAAA TTGTGATCTTTATTTCTAAAGAAATTATAAGAGTA TGCTTATATATACTGGAACTATTTCTTTTCTAATT | |
| | | TCCTGAAACCATTTGCAGGAAACTTAAGGATGGT CTATCTGAGAAGCAAAGGAGTCAGGAAGGCTGG AAAGGAGACTTATTTCTTGTTTTGTAAACTTACTG CTCTGTTTGAATTTACCCTAAGCATATATTATTTT TAGTTAGACATATAAGAAAATGTGTTTAACAATT CATTCATTTTTATCTTTGGTTTTGTCCATAGTATGT ATTTAGGAAGTTTCACAATGTGAAGAAAGCGAAG AGTAAAAACAGCAGGTGACTGCATTGACAAAAT GAAAGCAAGCAAATTGGACAAACCTAGCATAAA GAAGACAGACCAGGTTATGCATTTTTACTCCTTA CAAATATAAAGACAAGCCCGTCCTAAATTATCCC AGTAGAAAATTTTGACAACTACAACAAAACAGA AAACCCAACAAAACAAATAAAAATTTGATGCTG ATGACATGCGATAAAGAAAAGCAGAGCTCTCCA GTAAAAACCAAAATTCCTGTCTGTTAAGTTTACA AGGATTCTTCCTTAGGACTTTCTGAGCCAAGACA ATGGACAGCTAAGAGGGCAGCTCTTGAAATACTT TCACAGGATTTGCTTCTCTGCTCATAAAATGTAG GAACAATTCAAACTGGAGAGCATTTCCTACCCAT TCACTGGCCTGAAGATTCATAGAGTAGGGTTTTG TGTTGCTGTCAGCAGGCATTTATCTGAGCACACA TCCATCCATCCTCCCTCTCTCTCTCTCTCTCCTTCT CTCTCTCTCTCTTTCCCCCCACACCCGATGCATGC ATACACTCACACACTTTGGAACTTAGAAGGCCAC TCTTCTGAATATCAAATCAAGTAAAATGACCATT CTAAGGGTGACAACTCAGGGAAACTCTGGAAAC ACTGGAAACAAATACTGAAAATAAACTTACGTGT GGGAGAGAGAATAACATTGAAAGAAGGTATACA TAGTAACCAAAAGAGCTACTGTTTCATGTTTATA GGTTGGGAACTGAACAGAAAGTTGAAAACACAG CACACATGATTAAAAAATTTTTTCCTTAAAAACC AAAGAGCCATTTTTCTTAAATTCCTGATCAGATTT AATTAACTACATTTCAAATGCAGTCATGCACTGC ATAATGACATTTTGGTCAACAATGAACCATATCA GTGGTCCCACAGATTATAATGGAGCTGAAAAATT | |
| | | CTTATCATCTAGTGATGCTATAACGCCCGTACT | |
| LNCRNA3 | AC098484.1 | TAGTCAGAATCCACTACTCTACCACTAATGTCTCT TCCATCCGGCTCTGCAGCACCTGAGCAAAAGACC TCTGCAGCTGAGTCCAAAGAGCATTCCCATGTGA ACCGAGCAACCAAGTATCATCGGAGTGAGTTTGT TAGATTCTAGCCCCATCCTGTGCTAGGCCTTAGGT AGACCTCTTACAGCTCACAAGAAATGCAGTTTAT GAGGAAAGACCATGAATCGGGGGGCGGCCCGGG GAGGGTATTACCAAACAGATCAGCATCAGGAAC ATCATTCCCAGAGAGAAATTTGGCCAATTGTCAG TTCAAGTACACATCTCTCCCTCCTTTTACAGCATA TTGCAATAGATTATAGCCATGCGAAATAATCCAT AAACTTAGCTAATTCCACCAAACAGATAGGAAAG AAGGTAGAGTCCTACAAGAGCCTAGTCAGAATA AGAACTACAGTAATTTTGCCAAAACCCTGTTAAG TTCTTGAATAATTCTGGTCAAAATGGATTTCAATC TGAGTTAACTCACAGTCAACAATTTGGCTGTTTTT CTTGCTTCTCACTCTACTGCCTTACTCTCTTCCAC AGTGTTAAGTAGCCATTTAAATGGGTTTGTTCTGC TGCAGGTGGCTGAGCCTGGGATTTCCAACTTGGG AGACTACACAGTGGACTCTATAATGATTAGTGGC AGCATCCAATGTCTCTTCCAAGTCTAAAATTTGTG AGTTTCAAAGTTGACTCTCCCCAAACCTGGCCTTT GCTGCCTCAGATTCTGTCACTCTAGCAAGGCAGA GAGAGCAAAAGGGGCAAGACAAACCTCATCAAC AGCACAGAAATCCCAGAATCACACACTCTCTGGT CCAGCCAAGCCAAGTGTTTTTTTTTTTGTTGTTGT TGTTGTTTTTTAAATCACCCTCCATTACTCAACAT TCTCGTTCCCAGATGAATTTTAAACTGGAGTTGC ACCAACTGTGAGACAGCCT | 7 |
| LNCRNA4 | AC124283.1 | CTGGGTTCAAGATGCAGAATGGTGCCTACGGCA AAAAAAAAAAAAAAAAACACCCTCCTCCCTTCTTT ACCATTTGAATGGACATTTTCCTTACCTGTGATCC | 8 |
| | | CAACAGAAACAGATCCAGACCTATCATGTGAAGT CCACGTTCCAGGATCAGAAGTAACCAGTTTATGG ACTGAGCTTACACGGGAAAGTCTACCCCCGACTC CTTCTGGATAGTAACATACACAGCTGCATAAAAA CGTCTCCAAGGGGACATACGATGCATTTGCTTGG TGTCCCAGCCAAGCTCCCCACCGGCGACCTCACT GTTCCTTAGAGCTCAAGAGCTCGTCTCCTATCAAT CAGAGAACCCCATCAGCTGTGACCAACAGAGCTG GAGCCCTCTGTGGAGGGAGCTGACCCCACACACA GGACAGAGCAGAATCCTGATTATTTTACAAACTG CAAACCTTCTGAGTAAGAAGACAAAAATATACAT TCCAAGGTATCTGTAAAGTGCTTGGAAGATGCAG ACAGCTGCACCGAGGGGCTCTGATCCATCCACAC GCTGCGCTTTGCTGCGGTCACACACACGGTCTCA GTCACGTGATGGTTTTGCTTTTATTTCTTAAACGG CTGAGTGATAATCCAGCTAGTGTGCAGTCATTTC ATACCTTTCAATGGGCGTCACCGCAGTGACGCTG CCCCAGCCCCATGCTGAGGGCCGACACAATTCAC GGAACAGATTCATCATATTTGGTCTTTATGTAAAT AATAAATGTTTTAAAATTGCCTAAATATA | |

### REFERENCES

1. Lo Presti A et al., (2016), Molecular epidemiology, evolution and phylogeny of Chikungunya virus: an updating review. Infect. Genet. Evol. 2016, 41:270-278;
2. Brasilia: Ministry of Health, 2017. Brasilia. Ministério da Saúde. Secretaria de Vigilância em Saúde. Departamento de Vigilância das Doenças Transmissíveis. Chikungunya : manejo;
3. Morrison CR et al., (2016), Chikungunya virus: current perspectives on a reemerging virus. Microbiol. Spectr. 2016, 4:143-161;
4. Rodriguez-Morales AJ et al., (2016), Cardona-Ospina JA, Urbano-Garzon S, Sebastian Hurtado-Zapata J. Prevalence of Post-Chikungunya Infection Chronic Inflammatory Arthritis: A Systematic Review and Meta-Analysis. Arthritis Care Res (Hoboken). 2016 Dec;68(12):1849-1858;
5. Ng et al., (2009), II-1b, IL-6, and RANTES as Biomarkers of Chikungunya Severity. PLoS ONE 4(1): e4261;
6. Sepúlveda-Delgado et al., (2017), Inflammatory biomarkers, disease activity index, and self-reported disability may be predictors of chronic arthritis after chikungunya infection: brief report. Clinical rheumatology, 36(3), 695-699;
7. Enfasa et al., (2017): Hyperferritinemia is a potential marker of chronic chikungunya: A retrospective study on the Island of Curaçao during the 2014-2015 outbreak, Journal of Clinical Virology 86 31-38;
8. Anna Genaro et al., (2020), Ferritin, Erythrocyte Sedimentation Rate, and C-Reactive Protein Level in Patients with Chikungunya-Induced Chronic Polyarthritis. The American journal of tropical medicine and hygiene, 103(5), 2077-2082;
9. Putrada et al., (2019). Long term persistence of Chikungunya virus-associated manifestation and anti-chikungunya virus antibody in southern Thailand: 5 years of an outbreak in 2008-2009. Viral Immunology. 442-452, Vol 33, Number 2, 2020;
10. Gualberto Cavalcanti et al., (2019). IL-27 in patients with Chikungunya fever: A possible chronicity biomarker? Acta tropica, 196, 48-51;
11. Gualberto Cavalcanti et al., (2020). Increased serum levels of galectin-9 in patients with chikungunya fever. Virus research, 286, 198062;
12. Silva Ferreira et al., (2021). Biomarkers of severity and chronification in chikungunya fever: a systematic review and meta-analysis, Rev. Inst. Med. trop. S. Paulo. 2021;63:e16;
13. Diniz Lopes Marques et al., (2017), Recommendations of the Brazilian Society of Rheumatology for diagnosis and treatment of Chikungunya fever. Part 1 - Diagnosis and special situations. Rev Bras Reumatol Engl Ed 2017;57 Suppl 2:421-437;
14. Zhang, X., et al. (2019). The role of long noncoding RNA in major human disease. Bioorganic chemistry, 92, 103214;

## Claims

1. An *in vitro* method for predicting the risk of developing Post-Chikungunya Chronic Inflammatory Joint Disease (pCHIKV-CIJD) in a subject, said method comprising detecting and/or evaluating the expression of one or more biomarkers in a biological sample obtained from said subject, for example a blood sample, wherein said one or more biomarkers are selected from the following long non-coding RNA sequences identified below as SEQ ID NOs 1-4 or a variant sequence thereof having at least 95% identity with one of the sequences identified in SEQ ID NOs 1-4:
| Lnc-RNA# | Reference | SEQ ID NO: |
|---|---|---|
| LNCRNA1 | AC104561.4 | 1 |
| LNCRNA2 | AP000224.1 | 2 |
| LNCRNA3 | AC098484.1 | 3 |
| LNCRNA4 | AC124283.1 | 4 |

2. The method of Claim 1, wherein the evaluating step includes (a) quantifying the expression of said one or more selected biomarkers in a biological sample obtained from said patient to obtain an expression value for one or more of said biomarkers, and (b) comparing each expression value obtained at step (a) to a control value and (c) determining the risk of developing pCHIKV-CIJD based on said comparison.

3. The method of Claim 1 or 2, wherein said control value corresponds to average expression value of said biomarker as measured in symptomatic patients which do not develop pCHIKV-CIJD.

4. The method of any one of Claims 1-3, wherein a higher expression value of said one or more selected biomarker as compared to corresponding control value measured in control group of patients which do not develop pCHIKV-CIJD is predictive of a risk of developing pCHIKV-CIJD.

5. The method of any one of Claims 1-4, which comprises at least evaluating the expression of the long non-coding RNA of SEQ ID NO: 1 (LNCRNA1).

6. The method of Claim 5, further comprising evaluating the expression of the long non-coding RNA of SEQ ID NO:2 (LNCRNA2) , and; optionally, further evaluating the expression of the long non-coding RNA of SEQ ID NO:3 (LNCRNA3) or the expression of the long non-coding RNA of SEQ ID NO:4 (LNCRNA4).

7. The method according to any one of Claims 1 to 6, comprising extracting total RNA from the biological sample, wherein the expression of said one or more selected biomarkers is determined in the extracted total RNA.

8. The method according to any one of Claims 1 to 7, wherein the biological sample has been obtained from a subject within the acute or subacute phase of CHIKV infection, preferably within 2 to 30 days following the start of the first symptoms of chikungunya disease.

9. A kit comprising means for amplifying and/or detecting at least one sequence selected from the long non-coding RNA sequences identified in SEQ ID NOs:1-4, wherein the amplification and/or detection means allow the amplification, the detection and/or the quantification, of 1, 2, 3 or all 4 long non-coding RNA sequences in a biological sample.

10. The kit of Claim 9, wherein
(i) the amplification means comprises at least one pair of primers for specific amplification of one of the selected biomarkers sequences of SEQ ID NOs 1-4 or variant biomarker sequences having at least 95% identity to any of SEQ ID NOs: 1-4, each amplification primer comprising or consisting of a nucleotide sequence identical or complementary to a part of any of SEQ ID NOs:1-8, or to a variant sequence having at least 95% identity to any of SEQ ID NOs:1-8; or
(ii) the detection means comprises at least a nucleic acid probe whose nucleotide sequence comprises or consists of a complementary sequence to at least part of any of SEQ ID NOs:1-8, or to a variant sequence having at least 95% identity to any of SEQ ID NOs:1-8

11. The kit of Claim 9 or 10, comprising at least a nucleic acid probe for specifically detecting the biomarker LNCRNA1 of SEQ ID NO:1 or a variant sequence having at least 95% identity to SEQ ID NO:1 or SEQ ID NO:5, typically by quantitative detection.

12. The kit of Claim 9, 10 or 11, comprising at least a nucleic acid probe for specifically detecting the biomarker LNCRNA2 of SEQ ID NO:2 or a variant sequence having at least 95% identity to SEQ ID NO:2 or SEQ ID NO:6, typically by quantitative detection.

13. The kit of any one of Claims 9-12, comprising a nucleic acid probe for specifically detecting the biomarker LNCRNA3 of SEQ ID NO:3 or a variant sequence having at least 95% identity to SEQ ID NO:3 or SEQ ID NO:7, typically by quantitative detection.

14. The kit of any one of Claims 9-12, comprising at least a nucleic acid probe for specifically detecting the biomarker LNCRNA4 of SEQ ID NO:4 or a variant sequence having at least 95% identity to SEQ ID NO:4 or SEQ ID NO:8, typically by quantitative detection.

15. The kit of any of claims 9-14, for predicting the risk of developing Chronic Inflammatory Joint Disease Post-Chikungunya (pCHIKV-CIJD) in a subject.
